# EUROPEAN PATENT APPLICATION

(11) **EP 4 685 158 A1**
(43) Date of publication of application: **28.01.2026**
(21) Application number: 24190111.5
(22) Date of filing: 22.07.2024
(51) Int. Cl.: C07K 16/18, A61P 25/28, A61K 39/395, C07K 16/00, G01N 33/00, A61K 39/00

(54) **FIBRILLARY APOLIPOPROTEIN E (APOE) FOR USE IN A METHOD OF TREATMENT AND/OR PREVENTION OF A NEURODEGENERATIVE DISEASE**

(71) Applicant: Technische Universität München, 80333 München (DE)
(72) Inventor: The designation of the inventor has not yet been filed
(74) Representative: Engelhard, Markus

(57) **Abstract**

The present invention relates to the field of neurodegenerative diseases, in particular Alzheimer's disease. The present invention further relates to fibrillary Apolipoprotein E (ApoE) for use in a method of treatment and/or prevention of a neurodegenerative disease and methods of producing said fibrillary ApoE. Moreover, the present invention relates to an antigen-binding peptide specifically binding to fibrillary ApoE, preferably human ApoE, a method of generating said antigen-binding peptide, and its use in a method of treatment and/or prevention and/or diagnosis of a neurodegenerative disease in a patient in need thereof.

## Description

### FIELD OF THE INVENTION

The present invention relates to the field of neurodegenerative diseases, in particular Alzheimer's disease. The present invention further relates to fibrillary Apolipoprotein E (ApoE) for use in a method of treatment and/or prevention of a neurodegenerative disease and methods of producing said fibrillary ApoE. Moreover, the present invention relates to an antigen-binding peptide specifically binding to fibrillary ApoE, preferably human ApoE, a method of generating said antigen-binding peptide, and its use in a method of treatment and/or prevention and/or diagnosis of a neurodegenerative disease in a patient in need thereof.

### BACKGROUND OF THE INVENTION

Neurodegenerative diseases, such as Alzheimer's disease (AD) and Parkinson's disease are increasingly prevalent in aging populations worldwide. These debilitating conditions are characterized by the progressive loss of structure or function of neurons, leading to cognitive and motor impairments. As life expectancy rises, the incidence of neurodegenerative diseases is expected to grow, posing significant challenges for healthcare systems and emphasizing the urgent need for effective treatments and preventive strategies.

AD is preceded by a preclinical phase in which insoluble amyloid-β (Aβ) is deposited as extracellular plaques. This gradual build-up of amyloid **plaques** is thought to trigger a pathological cascade involving multiple cell types, eventually leading to neurodegeneration with progressive deterioration of memory and cognitive function. The seeded growth of self-replicating misfolded protein aggregates has served as a powerful conceptual framework in AD and other neurodegenerative diseases. But what exactly initiates this pathological cascade? Genetic analyses have shown that the risk of sporadic AD is linked to genes that are primarily expressed in microglia and astrocytes, which respond to the deposition of amyloid protein, but how these genes contribute to amyloidosis is not fully understood. The genetic determinants are found in immune, lipid and endocytic pathways, and among the identified susceptibility genes, Apolipoprotein E (*APOE*)*,* with its allelic isoforms *APOE4,* confers the greatest risk. Within the healthy central nervous system (CNS), APOE is mainly synthesized by astrocytes, and similar to other apolipoproteins, APOE functions by solubilizing the lipid load in the lipoprotein particle. Through interaction with different members of the low-density lipoprotein family, the lipidated APOE particles are delivered by receptor-mediated endocytosis into cells, where the lipids support processes, such as cell viability, synaptogenesis and membrane extension. In addition to its physiological role in lipid homeostasis, APOE plays a key role in neurological diseases. Recent studies have shown that virtually any injury to the CNS, including AD, results in microglia and astrocytes reactivity with an upregulation of lipid metabolic pathways and in particular APOE. APOE can capture and clear damaging lipids arising from cellular debris following tissue injury. For example, free cholesterol is toxic to cells, and needs to be transferred onto lipoprotein particles after acute CNS damage. While apolipoproteins have evolved to transport lipids, they can also protect against other damaging hydrophobic molecules, such as Aβ. However, in addition to the function of APOE in promoting clearance of Aβ and its aggregated forms, maladaptive and detrimental function can arise, as exemplified by the co-deposition of APOE with Aβ amyloid in the core of the plaques. Moreover, mouse knockout studies have shown that loss of APOE results in reduced fibrillary amyloid plaque deposition, indicating a role of APOE in Aβ fibrillization, stabilization of fibrillar Aβ and/or compaction of amyloid plaques.

The growing prevalence of Alzheimer's disease underscores the urgent need for new therapeutic interventions. Current treatments primarily offer symptomatic relief and do not halt or reverse the disease's progression, leaving a significant gap in effective management. As the global population ages, the burden of Alzheimer's disease on patients, caregivers, and healthcare systems intensifies, driving the necessity for innovative therapies that target the underlying mechanisms of the disease.

Emerging research highlights the complex interplay of genetic, environmental, and lifestyle factors in Alzheimer's pathogenesis, suggesting that multifaceted treatment approaches may be required. Novel therapeutic strategies, including disease-modifying drugs, immunotherapies, and gene therapies, are being explored to address the root causes of neuronal degeneration and amyloid plaque accumulation. Additionally, advancements in early diagnosis and personalized medicine hold promise for more effective and tailored interventions.

The development of new therapeutic means is not only crucial for improving outcomes and quality of life for those affected but also for alleviating the economic and social burdens associated with long-term care. Thus, there is a need for the identification of novel therapeutic targets for the treatment and/or prevention of neurodegenerative diseases, such as Alzheimer's disease. Moreover, there is an urgent need for novel means for the effective treatment and/or prevention of neurodegenerative diseases, in particular Alzheimer's disease.

### SUMMARY OF THE INVENTION

It is therefore an objective of the present invention to identify novel therapeutic targets for the treatment and/or prevention of neurodegenerative diseases, such as Alzheimer's disease. Moreover, it is an objective of the present invention to provide novel means for the effective treatment and/or prevention of neurodegenerative diseases, in particular Alzheimer's disease. It is further an objective of the present invention to provide methods for the generation of said novel means for the effective treatment and/or prevention of neurodegenerative diseases.

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a fibrillary Apolipoprotein E (ApoE) for use in a method of treatment and/or prevention of a neurodegenerative disease in a patient in need thereof;
wherein, preferably the fibrillary ApoE is a fibrillary recombinant ApoE, more preferably, a fibrillary recombinant human ApoE, most preferably a fibrillary recombinant human ApoE4.

In one embodiment, the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. 1.

In one embodiment, the ApoE is a fusion protein comprising one or more additional moieties or domains selected from a group comprising: affinity tags, linkers, and spacers, preferably, affinity tags and linkers, even more preferably affinity tags.

In one embodiment, the ApoE is a fusion protein comprising an affinity tag selected from a group comprising a polyhistidine-tag, such as His6-tag, Halo-tag, Flag-tag, 3x Flag-tag, HA-tag, 3x HA-tag, c-Myc-tag, V5-tag, Strep-tag II (8 amino acids, WSHPQFEK-tag; SEQ ID NO:35), polyarginine tag (9 amino acids, RRRRRRRRR; SEQ ID NO:36), streptavidin binding peptide (SBP; 9 amino acids, AWRHPQFGG; SEQ ID NO:37), cellulose-binding tag (36 amino acids; QQTVWGQCGG QGWSGPTSCV AGSACSTLNP YYAQCI; SEQ ID NO:38), spot tag (12 amino acids, PDRVRAVSHW SS; SEQ ID NO:39), C-tag (4 amino acids, EPEA; SEQ ID NO:40), ALFA-tag (13 amino acids, SRLEEELRRR LTE; SEQ ID NO:41), Avi-tag (15 amino acids, GLNDIFEAQK IEWHE; SEQ ID NO:42), chitin binding protein (CBP), maltose binding protein (MBP), and gluthathione-S-transferase (GST); preferably a polyhistidine-tag, such as His6-tag, Halo-tag, Flag-tag, 3x Flag-tag, HA-tag, 3x HA-tag, c-Myc-tag, and V5-tag, even more preferably a polyhistidine-tag, such as His6-tag, and Halo-tag; most preferably His6-tag;
wherein the affinity tag is fused to the N-terminus or the C-terminus of the ApoE sequence either directly or via a cleavable or non-cleavable linker, preferably the N-terminus.

In one embodiment, the ApoE is a fusion protein comprising an affinity tag, wherein the affinity tag comprises or consists of an amino acid sequence which is at least 90% identical, preferably at least 95% identical, more preferably at least 98% identical, even more preferably at least 99% identical, optionally identical, to an amino acid sequence selected from a group comprising SEQ ID No. 16, SEQ ID No. 17, SEQ ID No. 18, SEQ ID No. 19, SEQ ID No. 20, SEQ ID No. 21, SEQ ID No. 22, SEQ ID No. 23, and SEQ ID No. 24, preferably, SEQ ID No. 16 and SEQ ID No. 17, most preferably SEQ ID No. 16.

In one embodiment, the ApoE is a fusion protein comprising a linker, wherein the linker is either a amino acid-based linker, wherein the amino acid-based linker comprises or consists of an amino acid sequence which is at least 90% identical, preferably at least 95% identical, more preferably at least 98% identical, even more preferably at least 99% identical, optionally identical, to an amino acid sequence selected from a group comprising SEQ ID Nos. 25 to 34, or wherein the linker is a chemical linker, wherein the chemical linker is selected from a group comprising ValCitPABC linker, GGFG tetrapeptidyl-aminomethoxy linker, Aminoethyl-SS-ethylalcohol linker, Azido-Phenyl-Amido-S-S-Sulfo-NHS linker, Azidoethyl-SS-propionic NHS ester linker, Biotin-PEG4-S-S-acid linker, and Trifluoroacetamidoethyl-SS-propionic NHS ester linker.

In one embodiment, the ApoE is a fusion protein comprising a polyhistidine-tag, preferably His6-Tag, wherein the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. 2 or SEQ ID No. 3.

In one embodiment, the ApoE is a fusion protein comprising a Halo-tag, wherein the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. 4 or SEQ ID No. 5.

In one embodiment, the neurodegenerative disease is a neurodegenerative disease of the spectrum of central nervous system (CNS) diseases, more preferably, a neurodegenerative disease of the group of dementia conditions, even more preferably, a neurodegenerative diseases with an abnormal aggregation phenotype, even more preferably, an Alzheimer's disease such as early and late onset Alzheimer's disease, Alzheimer's disease including sporadic and/or familial cases, and Alzheimer's disease which occurs in conjunction with the presence of an ApoE4 allele.

In one embodiment, the fibrillary ApoE is produced in a method comprising the steps of:
i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence of SEQ ID No. 1, SEQ ID No. 2, SEQ ID No. 3, SEQ ID No. 4, and SEQ ID No. 5;
ii) cultivation of the suitable host cell under conditions that favor the production of the ApoE for an appropriate time period, wherein, optionally, the ApoE is secreted into the extracellular milieu;
iii) optionally, harvesting of the host cell(s);
iv) purification of the ApoE produced in step (ii);
v) optionally, lyophilization of the purified ApoE to obtain lyophilized ApoE;
vi) optionally, reconstitution of the lyophilized ApoE;
vii) optionally, separation of the ApoE according to their size;
viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL; most preferably to a concentration of about 500 µg/ml in TBS;
ix) inducing fibrillization of ApoE, optionally by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, EDTA, a buffer C, comprising NH₄HCO₃ (pH 7.8), and a buffer D, comprising NaCl, KCl, Na₂HPO₄, KH₂PO₄ (pH 7.4);
x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
xi) storage of the fibrillary ApoE at temperature in a range of about -20°C to about -80°C, preferably in a range of about -60°C to about -80°C, most preferably at about -80°C, until use.

In one preferred embodiment, the incubation in step x) is performed together with further components that enhance fibrillization; wherein said further components are selected from a group comprising:
isolated and/or immunopurified ApoE aggregates from mouse models in concentrations in a range from about 100 ng/mL to about 1000 ng/mL, more preferably in a range from about 200 ng/mL to about 500 ng, most preferably with about 500 ng/mL;
isolated and/or immunopurified ApoE aggregates from AD patients in concentrations in a range from about 10 ng/mL to about 100 ng/mL, more preferable in a range from about 20 ng to about 50 ng/mL, most preferable with about 50ng/mL; wherein the immopurified ApoE aggregates are derived from ApoE2/ApoE2 carriers, ApoE2/ApoE3 carriers, ApoE2/ApoE4 carriers ApoE3/ApoE3 carriers, ApoE3/ApoE4 carriers, and/or ApoE4/ApoE4 carriers, isolated using sarkosyl, preferentially 1% to 3%, most preferably 2% sarkosyl;
isolated beta-sheet dye positive aggregated material from cells treated with recombinant APOE;
cell lysate from cells harboring beta-sheet dye positive fibrils after treatment with recombinant APOE;
beads, such as agarose beads, metal beads, or glass beads;
ApoE fibrils attached to said beads; and/or
combinations thereof.

In a preferred embodiment, when buffer A is used in step ix), the final concentration comprises about 9.1 mM Tris, 260 mM NaCl, 0.22 mM EDTA, 2.5 mM KCl, 9.1 mM Na₂HPO₄, 1.8 mM KH₂PO₄ (pH 7.4).

In a preferred embodiment, when buffer B is used in step ix), the final concentration comprises about 10 mM Tris, 150 mM NaCl, 0.25 mM EDTA (pH 7.4).

In a preferred embodiment, when buffer C is used in step ix), the final concentration comprises about 100 mM NH₄HCO₃ (pH 7.8).

In a preferred embodiment, when buffer D is used in step ix), the final concentration comprises about 135 mM NaCl, 2.7 mM KCl, 4.3 mM Na₂HPO₄, 1.4 mM KH₂PO₄ (pH 7.4).

In one preferred embodiment, the incubation step x) is performed under conditions favouring fibrillization, such conditions being selected from an increased salt concentration and/or a decreased pH. With respect to such "increased salt concentration" and "decreased pH", the point of reference is a regular TBS buffer, for which typical salt concentrations lie in the range of from 100 mM to 200 mM, and for which typical pH values lie in the range of from 7.2 to 8.0. Hence, accordingly, in one embodiment, an "increased salt concentration" is a salt concentration, in particular a concentration of NaCl, that is > 200mM, e.g. 210 mM, 220 mM, 230 mM, 240 mM, 250 mM, 260 mM, 270 mM, 280 mM, 290 mM or 300 mM and any concentrations in between 200mM and 300mM or even greater than 300mM. Likewise, in one embodiment, a decreased pH is a pH that is < 7.2, e.g. 7.0, 6.9, 6.8, 6.7, 6.6, 6.5, 6.4, 6.3, 6.2, 6.1, 6.0, and values even below 6.0.

In one embodiment, the suitable host cell is selected from a group comprising microorganisms, such as bacteria or yeasts; mammalian cells; human cells; animal cells; and animals; wherein, preferably, the suitable host cell is selected from bacteria, yeasts, and animal cells; more preferably, bacteria, and animal cells; even more preferably selected from an *Escherichia coli* strain, a HEK cell line, and a CHO cell line; wherein, most preferably, the suitable host cell is *E. coli* BL21.

In one embodiment, the method of treatment and/or prevention comprises administering the recombinant ApoE to a patient in an amount sufficient to induce an immune response; wherein, preferably, the immune response comprises the production of antibodies specifically binding to fibrillary ApoE, preferably fibrillary human ApoE4, by the patient.

In one embodiment, the patient is a human or an animal, preferably a human, even more preferably a human expressing ApoE4.

In a further aspect, the present invention also relates to a fibrillary Apolipoprotein E (ApoE) for use in therapy of a patient;
wherein, preferably the fibrillary ApoE is a fibrillary recombinant ApoE, more preferably, a fibrillary recombinant human ApoE, most preferably a fibrillary recombinant human ApoE4. In this aspect, the ApoE and the fibrillary ApoE are preferably as defined further above and as defined herein.

In another aspect, the present invention relates to an antigen-binding peptide specifically binding to fibrillary human ApoE, wherein the fibrillary human ApoE consists of an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 6, and SEQ ID No. 11, preferably SEQ ID No. 1.

In one embodiment, the antigen-binding peptide does not specifically bind to lipidated states of ApoE, such as lipoproteins; and/or
the antigen-binding peptide does not specifically bind to non-fibrillary conformational states of ApoE, such as monomeric ApoE, dimeric ApoE, tetrameric ApoE, or octameric ApoE.

In one embodiment, the antigen-binding peptide is produced by a method comprising the steps of:
(a) generating fibrillary ApoE by a method comprising the steps:
   i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, preferably SEQ ID No.1, SEQ ID No. 2, SEQ ID No. 3, SEQ ID No. 4, and SEQ ID No. 5;
   ii) cultivation of the suitable host cell under conditions that favor the production of the ApoE for an appropriate time period, wherein, optionally, the ApoE is secreted into the extracellular milieu;
   iii) optionally, harvesting of the host cell(s);
   iv) purification of the ApoE produced in step (ii) and/or (iii);
   v) optionally, lyophilization of the purified ApoE to obtain lyophilized ApoE;
   vi) optionally, reconstitution of the lyophilized ApoE;
   vii) optionally, separation of the ApoE according to their size;
   viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL, most preferably to a concentration of about 500 µg/ml in TBS;
   ix) inducing fibrillization of ApoE by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, and EDTA, a buffer C, comprising NH₄HCO₃ (pH 7.8), and a buffer D, comprising NaCl, KCl, Na₂HPO₄, KH₂PO₄ (pH 7.4);
   x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
   xi) storage of the fibrillary ApoE at temperature in a range of about -20°C to about
      - 80°C, preferably in a range of about -60°C to about -80°C, most preferably at about -80°C, until use;
(b) administering the fibrillary ApoE, preferably ApoE in Buffer A, obtained in step (a) to a suitable animal host in an amount sufficient to induce an immune response; wherein, preferably, the immune response comprises the production of antibodies specifically binding to fibrillary ApoE, preferably fibrillary human ApoE, even more preferably to fibrillary human ApoE4;
   wherein, the suitable animal host is selected from a group comprising rodents, mice, rats, rabbits, chickens, sheep, guinea pigs, goats, donkey, horses and camelids; preferably selected from mice, rats, and rabbits; more preferably selected from mice and rats; even more preferably selected from wistar rats and CD1 mice;
   wherein, preferably, the fibrillary ApoE is administered subcutaneously (s.c.) and/or intraperitoneally (i.p.);
   wherein, optionally, the fibrillary ApoE is administered together with one or more further adjuvants, such as cytidine-phosphate-guanosine or incomplete Freud's adjuvant;
(c) keeping the animal host for a time period of about 5 weeks to about 20 weeks, preferably for about 8 weeks to about 17 weeks, more preferably for about 10 weeks to about 14 weeks, most preferably for about 12 weeks;
(d) optionally, during keeping in step (c), subsequent administering, preferably subcutaneously (s.c.) and/or intraperitoneally (i.p.) administering, of fibrillary ApoE about 1-7 days, preferably about 2-5 days, most preferably about 3 days before the end of keeping;
   wherein, optionally, the fibrillary ApoE is administered together with one or more further adjuvants; wherein, preferably, the subsequent administering is without Freud's adjuvant;
(e) isolating immune cells from the spleen of immunized animals generated in steps (a) - (d) and fusion of said immune cells with an immortalized myeloma cell line, preferably myeloma cell line P3X63-Ag.653, to generate hybridoma cells;
(f) incubating generated hybridoma cells of step (e) for an appropriate time period followed by screening of the hybridoma cells to identify anti-fibrillary-ApoE antigen-binding peptide producing hybridoma cells;
(g) subcloning by limiting dilution of anti-fibrillary-ApoE antigen-binding peptide producing hybridoma cells to obtain stable monoclonal cell lines;
(h) cultivating the stable monoclonal cell lines obtained in step (g) under conditions suitable for the production of the anti-fibrillary-ApoE antigen-binding peptide;
(i) isolation, purification, and storage of the anti-fibrillary-ApoE antigen-binding peptide produced in step (h).

In one embodiment, the antigen-binding peptide is produced by a method comprising the steps of:
(a) generating fibrillary ApoE by a method comprising the steps:
   i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, preferably SEQ ID No.1, SEQ ID No. 2, SEQ ID No. 3, SEQ ID No. 4, and SEQ ID No. 5;
   ii) cultivation of the suitable host cell under conditions that favor the production of the ApoE for an appropriate time period, wherein, optionally, the ApoE is secreted into the extracellular milieu;
   iii) optionally, harvesting of the host cells;
   iv) purification of the ApoE produced in step (ii) and/or (iii);
   v) optionally, lyophilization of the purified ApoE to obtain lyophilized ApoE;
   vi) optionally, reconstitution of the lyophilized ApoE;
   vii) optionally, separation of the ApoE according to their size;
   viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL, most preferably to a concentration of about 500 µg/ml in TBS;
   ix) inducing fibrillization of ApoE by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, and EDTA, a buffer C, comprising NH₄HCO₃ (pH 7.8), and a buffer D, comprising NaCl, KCl, Na₂HPO₄, KH₂PO₄ (pH 7.4);
   x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
   xi) storage of the fibrillary ApoE at temperature in a range of about -20°C to about -80°C, preferably in a range of about -60°C to about -80°C, most preferably at about -80°C, until use;
(b) administering the fibrillary ApoE, preferably ApoE in Buffer A, obtained in step (a) to a suitable animal host in an amount sufficient to induce an immune response; wherein, preferably, the immune response comprises the production of antibodies specifically binding to fibrillary ApoE, preferably fibrillary human ApoE, even more preferably to fibrillary human ApoE4;
   wherein, the suitable animal host is selected from a group comprising rodents, mice, rats, rabbits, chickens, sheep, guinea pigs, goats, donkey, horses and camelids; preferably selected from mice, rats, and rabbits; more preferably selected from mice and rats; even more preferably selected from wistar rats and CD1 mice;
   wherein, preferably, the fibrillary ApoE is administered subcutaneously (s.c.) and/or intraperitoneally (i.p.);
   wherein, optionally, the fibrillary ApoE is administered together with one or more further adjuvants, such as cytidine-phosphate-guanosine or incomplete Freud's adjuvant;
(c) boosting the immune system of the animal host by applying pertussis toxin intraperitoneally (i.p.) on the day of immunization and 2 days later to enhance the immune reaction;
(d) keeping the animal host for a time period of about 5 weeks to about 20 weeks, preferably for about 8 weeks to about 17 weeks, more preferably for about 10 weeks to about 14 weeks, most preferably for about 12 weeks;
(e) optionally, during keeping in step (d), subsequent administering, preferably subcutaneously (s.c.) and/or intraperitoneally (i.p.) administering, of fibrillary ApoE about 1-7 days, preferably about 2-5 days, most preferably about 3 days before the end of keeping;
   wherein, optionally, the fibrillary ApoE is administered together with one or more further adjuvants; wherein, preferably, the subsequent administering is without Freud's adjuvant;
(f) isolation, purification, and storage of the anti-fibrillary-ApoE antigen-binding peptide, preferably an antibody, from the blood obtained from immunized animals generated in steps (a) - (e).

In one preferred embodiment, the incubation in step x) is performed together with further components that enhance fibrillization; wherein said further components are selected from a group comprising:
isolated and/or immunopurified APOE aggregates from mouse models in concentrations in a range from about 100 ng/mL to about 1000 ng/mL, preferably in a range from about 200 ng/mL to about 500 ng/mL, morepreferably with about 500 ng/mL;
isolated and/or immunopurified APOE aggregates from AD patients in concentrations in a range from about 10 ng/mL to about 100 ng/mL, more preferable in a range from about 20 ng/mL to about 50 ng/mL, most preferable with about 50 ng/mL; wherein the immopurified ApoE aggregates are derived from ApoE2/ApoE2 carriers, ApoE2/ApoE3 carriers, ApoE2/ApoE4 carriers ApoE3/ApoE3 carriers, ApoE3/ApoE4 carriers, and/or ApoE4/ApoE4 carriers, isolated using sarkosyl, preferentially 1% to 3%, most preferably 2% sarkosyl;
isolated beta-sheet dye positive aggregated material from cells treated with recombinant APOE;
cell lysate from cells harboring beta-sheet dye positive fibrils after treatment with recombinant APOE;
beads, such as agarose beads, metal beads, or glass beads;
ApoE fibrils attached to said beads; and/or
combinations thereof.

In one preferred embodiment, the incubation step x) is performed under conditions favouring fibrillization, such conditions being selected from an increased salt concentration and/or a decreased pH. With respect to such "increased salt concentration" and "decreased pH", the point of reference is a regular TBS buffer, for which typical salt concentrations lie in the range of from 100 mM to 200 mM, and for which typical pH values lie in the range of from 7.2 to 8.0. Hence, accordingly, in one embodiment, an "increased salt concentration" is a salt concentration, in particular a concentration of NaCl, that is > 200mM, e.g. 210 mM, 220 mM, 230 mM, 240 mM, 250 mM, 260 mM, 270 mM, 280 mM, 290 mM or 300 mM and any concentrations in between 200mM and 300mM or even greater than 300mM. Likewise, in one embodiment, a decreased pH is a pH that is < 7.2, e.g. 7.0, 6.9, 6.8, 6.7, 6.6, 6.5, 6.4, 6.3, 6.2, 6.1, 6.0, and values even below 6.0.

In one embodiment, said antigen-binding peptide is a human antibody, a humanized antibody, or a chimeric-human antibody;
wherein, optionally, said antigen-binding peptide is an intact antibody, a substantially intact antibody, a Fab fragment, a F(ab')₂ fragment, or a single chain Fv fragment.

In one embodiment said antigen-binding peptide is further modified.

In one embodiment, said modifications include Fab fragment modifications such as to achieve pH-dependent binding antibodies e.g. by direct mutagenesis of histidine residues into complementary determining regions (CDRs) or the framework region (FR). These antibodies have the potential to be recycled and shown to reduce relapse.

In one embodiment, said modifications include bispecific antibody modification to increase blood brain barrier penetration or the fusion of the antigen-biding peptide, for example an IgG antibody, to a second affinity ligand targeting endothelial cells for easier passing the blood brain barrier and thus, higher availability in the brain. Possible second affinity ligands, also referred to as shuttles, to fuse to the antigen-binding peptide include the transferrin receptor or CD98hc.

In one embodiment, said modification include the generation of single-domain brain-targeting antibody fragments, such as nanobodies from llamas.

In a preferred embodiment, when buffer A is used in step ix), the final concentration comprises about 9.1 mM Tris, 260 mM NaCl, 0.22 mM EDTA, 2.5 mM KCl, 9.1 mM Na₂HPO₄, 1.8 mM KH₂PO₄ (pH 7.4).

In a preferred embodiment, when buffer B is used in step ix), the final concentration comprises about 10 mM Tris, 150 mM NaCl, 0.25 mM EDTA (pH 7.4).

In a preferred embodiment, when buffer C is used in step ix), the final concentration comprises about 100 mM NH₄HCO₃ (pH 7.8).

In a preferred embodiment, when buffer D is used in step ix), the final concentration comprises about 135 mM NaCl, 2.7 mM KCl, 4.3 mM Na₂HPO₄, 1.4 mM KH₂PO₄ (pH 7.4).

In one embodiment, the antigen-binding peptide is capable of crossing the blood-brain barrier.

In another aspect, the present invention relates to a method for generating an antigen-binding peptide specifically binding to fibrillary ApoE, preferably fibrillary ApoE4, the method comprising the steps of:
(a) generating fibrillary ApoE by a method comprising the steps:
   i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, preferably SEQ ID No.1, SEQ ID No. 2, SEQ ID No. 3, SEQ ID No. 4, and SEQ ID No. 5;
   ii) cultivation of the suitable host cell under conditions that favor the production of the ApoE for an appropriate time period, wherein, optionally, the ApoE is secreted into the extracellular milieu;
   iii) optionally, harvesting of the host cells;
   iv) purification of the ApoE produced in step (ii) and/or (iii);
   v) optionally, lyophilization of the purified ApoE to obtain lyophilized ApoE;
   vi) optionally, reconstitution of the lyophilized ApoE;
   vii) optionally, separation of the ApoE according to their size;
   viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL, most preferably to a concentration of about 500 µg/ml in TBS;
   ix) inducing fibrillization of ApoE by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, EDTA, a buffer C, comprising NH₄HCO₃ (pH 7.8), and a buffer D, comprising NaCl, KCl, Na₂HPO₄, KH₂PO₄ (pH 7.4);
   x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
   xi) storage of the fibrillary ApoE at temperature in a range of about -20°C to about -80°C, preferably in a range of about -60°C to about -80°C, most preferably at about -80°C, until use;
(b) administering the fibrillary ApoE in Buffer A obtained in step (a) to a suitable animal host in an amount sufficient to induce an immune response; wherein, preferably, the immune response comprises the production of antibodies specifically binding to fibrillary ApoE, preferably fibrillary human ApoE, even more preferably to fibrillary human ApoE4;
   wherein, the suitable animal host is selected from a group comprising rodents, mice, rats, rabbits, chickens, sheep, guinea pigs, goats, donkey, horses, and camelids; preferably selected from mice, rats, and rabbits; more preferably selected from mice and rats; even more preferably selected from wistar rats and CD1 mice;
   wherein, preferably, the fibrillary ApoE in Buffer A is administered subcutaneously (s.c.) and/or intraperitoneally (i.p.);
   wherein, optionally, the fibrillary ApoE is administered together with one or more further adjuvants, such as cytidine-phosphate-guanosine and incomplete Freud's adjuvant;
(c) keeping the animal host for a time period of about 5 weeks to about 20 weeks, preferably for about 8 weeks to about 17 weeks, more preferably for about 10 weeks to about 14 weeks, most preferably for about 12 weeks;
(d) optionally, during keeping in step (c), subsequent administering, preferably subcutaneously (s.c.) and/or intraperitoneally (i.p.) administering, of fibrillary ApoE about 1-7 days, preferably about 2-5 days, most preferably about 3 days before the end of keeping;
   wherein, optionally, the fibrillary ApoE is administered together with one or more further adjuvants; wherein, preferably, the subsequent administering is without Freud's adjuvant;
(e) isolating immune cells from the spleen of immunized animals generated in steps (a) - (d) and fusion of said immune cells with an immortalized myeloma cell line, preferably myeloma cell line P3X63-Ag.653, to generate hybridoma cells;
(f) incubating generated hybridoma cells of step (e) for an appropriate time period followed by screening of the hybridoma cells to identify anti-fibrillary-ApoE antigen-binding peptide producing hybridoma cells;
(g) subcloning by limiting dilution of anti-fibrillary-ApoE antigen-binding peptide producing hybridoma cells to obtain stable monoclonal cell lines;
(h) cultivating the stable monoclonal cell lines obtained in step (g) under conditions suitable for the production of the anti-fibrillary-ApoE antigen-binding peptide;
(i) isolation, purification, and storage of the anti-fibrillary-ApoE antigen-binding peptide produced in step (h).

In one preferred embodiment, the incubation in step x) is performed together with further components that enhance fibrillization; wherein said further components are selected from a group comprising:
isolated and/or immunopurified APOE aggregates from mouse models in concentrations in a range from about 100 ng/mL to about 1000 ng/mL, preferably in a range from about 200 ng/mL to about 500 ng/mL, more preferably with 500 ng/mL;
isolated and/or immunopurified APOE aggregates from AD patients in concentrations in a range from about 10 ng/mL to about 100 ng/mL, more preferable in a range from about 20 ng/mL to about 50 ng/mL, most preferable with about 50 ng/mL; wherein the immopurified ApoE aggregates are derived from ApoE2/ApoE2 carriers, ApoE2/ApoE3 carriers, ApoE2/ApoE4 carriers ApoE3/ApoE3 carriers, ApoE3/ApoE4 carriers, and/or ApoE4/ApoE4 carriers, isolated using sarkosyl, preferentially 1% to 3%, most preferably 2% sarkosyl;
isolated beta-sheet dye positive aggregated material from cells treated with recombinant APOE;
cell lysate from cells harboring beta-sheet dye positive fibrils after treatment with recombinant APOE;
beads, such as agarose beads, metal beads, or glass beads;
ApoE fibrils attached to said beads; and/or
combinations thereof.

In a preferred embodiment, when buffer A is used in step ix), the final concentration comprises about 9.1 mM Tris, 260 mM NaCl, 0.22 mM EDTA, 2.5 mM KCl, 9.1 mM Na₂HPO₄, 1.8 mM KH₂PO₄ (pH 7.4).

In a preferred embodiment, when buffer B is used in step ix), the final concentration comprises about 10 mM Tris, 150 mM NaCl, 0.25 mM EDTA (pH 7.4).

In a preferred embodiment, when buffer C is used in step ix), the final concentration comprises about 100 mM NH₄HCO₃ (pH 7.8).

In a preferred embodiment, when buffer D is used in step ix), the final concentration comprises about 135 mM NaCl, 2.7 mM KCl, 4.3 mM Na₂HPO₄, 1.4 mM KH₂PO₄ (pH 7.4).

In one preferred embodiment, the incubation step x) is performed under conditions favouring fibrillization, such conditions being selected from an increased salt concentration and/or a decreased pH. With respect to such "increased salt concentration" and "decreased pH", the point of reference is a regular TBS buffer, for which typical salt concentrations lie in the range of from 100 mM to 200 mM, and for which typical pH values lie in the range of from 7.2 to 8.0. Hence, accordingly, in one embodiment, an "increased salt concentration" is a salt concentration, in particular a concentration of NaCl, that is > 200mM, e.g. 210 mM, 220 mM, 230 mM, 240 mM, 250 mM, 260 mM, 270 mM, 280 mM, 290 mM or 300 mM and any concentrations in between 200mM and 300mM or even greater than 300mM. Likewise, in one embodiment, a decreased pH is a pH that is < 7.2, e.g. 7.0, 6.9, 6.8, 6.7, 6.6, 6.5, 6.4, 6.3, 6.2, 6.1, 6.0, and values even below 6.0.

In one aspect, the present invention relates to an antigen-binding peptide generated by a method according to the foregoing aspect.

In one embodiment, the present invention relates to the antigen-binding peptide as defined in any of the above embodiments, for use in a method of treatment and/or prevention and/or diagnosis of a neurodegenerative disease in a patient in need thereof; wherein, preferably, the neurodegenerative disease is a neurodegenerative disease of the spectrum of central nervous system (CNS) diseases, more preferably, a neurodegenerative disease of the group of dementia conditions, even more preferably, a neurodegenerative diseases with an abnormal aggregation phenotype, even more preferably, an Alzheimer's disease such as early and late onset Alzheimer's disease, Alzheimer's disease including sporadic and/or familial cases, and Alzheimer's disease which occurs in conjunction with the presence of an ApoE4 allele.

In one aspect, the present invention relates to a composition, preferably a pharmaceutical composition, comprising a fibrillary ApoE for use according to any of the above embodiments, or an antigen-binding peptide according to any of the above embodiments.

In one embodiment, said composition further comprises at least one pharmaceutically acceptable carrier, diluent and/or excipient.

In one embodiment, the present invention relates to a composition as defined in the above embodiments for use in a method of treatment and/or prevention of a neurodegenerative disease in a patient in need thereof; wherein, preferably, the neurodegenerative disease is a neurodegenerative disease of the spectrum of central nervous system (CNS) diseases, more preferably, a neurodegenerative disease of the group of dementia conditions, even more preferably, a neurodegenerative diseases with an abnormal aggregation phenotype, even more preferably, an Alzheimer's disease such as early and late onset Alzheimer's disease, Alzheimer's disease including sporadic and/or familial cases, and Alzheimer' s disease which occurs in conjunction with the presence of an ApoE4 allele.

In one embodiment of the fibrillary ApoE for use as defined herein or of the antigen-binding peptide for use as defined herein or of the composition for use as defined herein, the fibrillary ApoE, the antigen-binding peptide, or the composition is administered to a patient in need thereof.

In one embodiment relating to the composition for use, the antigen-binding peptide, or the composition is administered to a patient in combination with one or more further therapies; wherein, preferably, the one or more further therapies are selected from Abeta-targeting therapies, such as a therapy with Aducanumab, and anti-inflammatory therapies, such as a therapy with one or more STAT/JAK inhibitors.

In one embodiment, the method of diagnosis of a neurodegenerative disease is a method of diagnosis of ApoE fibrillization in a biological sample derived from a patient; wherein, preferably, the biological sample is serum or cerebrospinal fluid; wherein, preferably, said method of diagnosis is performed during early stages of an Alzheimer's disease, such as preclinical Alzheimer's disease (biomarker positive), and the stage of mild cognitive impairment.

In one embodiment, the patient is a human or an animal, preferably a human, even more preferably a human expressing ApoE4.

Another aspect of the present application relates to a method of treatment and/or prevention of a neurodegenerative disease in a patient in need thereof, wherein said method comprises administering a fibrillary Apolipoprotein E (ApoE) and/or a pharmaceutical composition comprising said fibrillary ApoE to said patient; wherein, preferably the fibrillary ApoE is a fibrillary recombinant ApoE, more preferably, a fibrillary recombinant human ApoE, most preferably a fibrillary recombinant human ApoE4.

According to this aspect, the fibrillary ApoE, the pharmaceutical composition, the patient, and the neurodegenerative disease are as defined herein.

Another aspect of the present application relates to a method of treatment and/or prevention of a neurodegenerative disease in a patient in need thereof, wherein said method comprises administering an antigen-binding peptide specifically binding to fibrillary human ApoE and/or a pharmaceutical composition comprising said antigen-binding peptide.

According to this aspect, the antigen-binding peptide, the pharmaceutical composition, the patient, and the neurodegenerative disease are as defined herein.

Another aspect of the present invention relates to the use of a fibrillary Apolipoprotein E (ApoE) and/or a pharmaceutical composition comprising said fibrillary ApoE for the manufacture of a medicament for the treatment and/or prevention of a neurodegenerative disease in a patient in need thereof; wherein, preferably the fibrillary ApoE is a fibrillary recombinant ApoE, more preferably, a fibrillary recombinant human ApoE, most preferably a fibrillary recombinant human ApoE4.

According to this aspect, the fibrillary ApoE, the pharmaceutical composition, the patient, and the neurodegenerative disease are as defined herein.

Another aspect of the present invention relates to the use of an antigen-binding peptide specifically binding to fibrillary human ApoE and/or a pharmaceutical composition comprising said antigen-binding peptide for the manufacture of a medicament for the treatment and/or prevention of a neurodegenerative disease in a patient in need thereof.

According to this aspect, the antigen-binding peptide, the pharmaceutical composition, the patient, and the neurodegenerative disease are as defined herein.

### DETAILED DESCRIPTION

In one embodiment, the term "patient" relates to a human or an animal, preferably a human.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

The term "Apolipoprotein E", short "ApoE", as used herein, refers to a protein involved in the metabolism of fats in the body of mammals and belongs to the family of fat-binding proteins called apolipoproteins. ApoE is mainly synthesized by astrocytes, and similar to other apolipoproteins, ApoE functions by solubilizing the lipid load in the lipoprotein particle. Through interaction with different members of the low-density lipoprotein family, the lipidated ApoE particles are delivered by receptor-mediated endocytosis into cells, where the lipids support processes, such as cell viability, synaptogenesis and membrane extension. In addition to its physiological role in lipid homeostasis, ApoE plays a key role in neurological diseases. Different alleles of ApoE exist. Humans have three major alleles of the APOE gene, designated as ApoE2, ApoE3, and ApoE4 (also referred to as ApoE ε2, ApoE ε3, and ApoE ε4), wherein each human may inherit two of the three ApoE alleles. The alleles differ by single amino acid changes: ApoE2 has cysteines at positions 112 and 158, ApoE3 has an arginine at position 158, and ApoE4 has arginines at both positions 112 and 158. ApoE associates with lipids to form lipoprotein particles, which is also referred to as lipidated ApoE. Lipid-free ApoE is referred to as unlipidated ApoE.

The term "fibrillary ApoE", as used herein, refers to aggregated ApoE which is insoluble to detergents or detergent containing buffers, such as RIPA buffer, partially adopts β-sheet secondary structure and stains positive with dyes such as Methoxy-X04, ThiosineRed and Thioflavin. When analyzed with electron microscopy, fibrillary ApoE appears as snake like and/or wavy fibrillar structures. Fibrillary ApoE may be formed by ApoE2, ApoE3, ApoE4 or mixtures thereof. Fibrillary ApoE may consist of natural or/or recombinant ApoE.

Fibrillization of ApoE to form ApoE fibrils can be induced by adjusting the concentration of ApoE, preferably monomeric ApoE, to a concentration in the range of about 20 µg/mL to about 3000 µg/mL in TBS, preferably about 200 µg/mL to about 700 µg/mL; most preferably in a concentration of about 500 µg/ml in TBS;
inducing fibrillization of ApoE, optionally by the addition of one or more components; wherein, preferably, said components are selected from a group comprising a buffer A, comprising Tris, NaCl, EDTA, KCl, Na₂HPO₄, and KH₂PO₄ (pH 7.4)10% of 10xPBS, a buffer B, comprising 10 mM Tris, 150 mM NaCl, 0.25 mM EDTA, a buffer C, comprising 100 mM NH₄HCO₃ (pH 7.8), and a buffer D, comprising 135 mM NaCl, 2.7 mM KCl, 4.3 mM Na₂HPO₄, 1.4 mM KH₂PO₄ (pH 7.4);
incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks.

Preferably, said fibrilization of ApoE is performed together with further components that enhance fibrillization; wherein said further components are selected from a group comprising: isolated and/or immunopurified APOE aggregates from mouse models in concentrations in a range from about 100 ng/mL to about 1000 ng/mL, preferably in a range from about 200 ng/mL to about 500 ng/mL, more preferably with about 500 ng/mL;
isolated and/or immunopurified APOE aggregates from AD patients in concentrations in a range from about 10 ng/mL to about 100 ng/mL, more preferable in a range from about 20 ng/mL to about 50 ng/mL, most preferable with about 50 ng/mL; wherein the immopurified ApoE aggregates are derived from ApoE2/ApoE2 carriers, ApoE2/ApoE3 carriers, ApoE2/ApoE4 carriers ApoE3/ApoE3 carriers, ApoE3/ApoE4 carriers, and/or ApoE4/ApoE4 carriers, isolated using sarkosyl, preferentially 1% to 3%, most preferably 2% sarkosyl;
isolated beta-sheet dye positive aggregated material from cells treated with recombinant ApoE;
cell lysate from cells harboring beta-sheet dye positive fibrils after treatment with recombinant APOE;
beads, such as agarose beads, metal beads, or glass beads;
ApoE fibrils attached to said beads; and/or
combinations thereof.

For the present disclosure the term also refers to recombinant variants of the protein including fusion proteins which may comprise one or more additional moieties or domains selected from a group comprising: affinity tags, linkers, and spacers, preferably, affinity tags and linkers, even more preferably affinity tags.

In one embodiment of the fibrillary ApoE for use, such fibrillary ApoE comprises or consist of a naturally occurring or recombinant ApoE that is present in aggregated fibrillary form. In one embodiment said ApoE comprises or consists of an amino acid sequence which is at least 90% identical, preferably at least 95% identical, more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, and SEQ ID No. 5.

The term "Alzheimer's disease", as used herein, refers to a progressive neurodegenerative disorder characterized by the gradual loss of cognitive function, memory, and the ability to perform daily activities. It is the most common cause of dementia in older adults. The disease is marked by the accumulation of amyloid-beta plaques and neurofibrillary tangles composed of tau protein in the brain, leading to neuronal damage and brain atrophy. Symptoms typically begin with mild memory loss and confusion and progressively worsen to include severe cognitive and behavioral impairments.

Cases of Alzheimer's disease include sporadic and or familial cases, wherein Alzheimer's disease can also occur in conjunction with the presence of an ApoE4 allele in a subject. For the present application, the term "sporadic Alzheimer's disease" or "sporadic cases of Alzheimer's disease" refers to refers to a form of Alzheimer's disease that typically develops in individuals aged 65 or older, and with the ApoE ε4 allele as the most important genetic risk factor. Moreover, the term "familial Alzheimer's disease" or "familial cases of Alzheimer's disease" refers to refers to a hereditary condition characterized by symptoms appearing at an unusually early age, typically before the age of 65. This form of Alzheimer's disease is often caused by mutations in the presenilin 1, presenilin 2, or amyloid precursor protein genes, which are inherited in an autosomal dominant pattern. The term "Alzheimer's disease" further encompasses early stages of an Alzheimer's disease, such as preclinical Alzheimer's disease (biomarker positive), and the stage of mild cognitive impairment.

The term "beta-sheet dye positive", as used herein refers to protein structures that can be stained with certain dyes, called β-sheet-dyes. These are a group of dyes that recognize a specific form of protein secondary structure namely β-sheet or β-plated-sheet, which consist of a configuration of multiple parallel or anti-parallel connected β-strands stabilized by hydrogen bonds. These dyes have certain characteristics to visualize β-sheet structures by electrostatically-driven adsorption, specific binding affinity preferring β-sheet structures over other configurations, immobilization by the fibrillary structure and thereby loosing the quenching ability of photon excitation.

In one embodiment, the patient is a human in a preclinical Alzheimer's disease state.

The term "preclinical stages of Alzheimer's disease", as used herein is conceptualized as a continuum that begins long before the onset of clinical symptoms. These stages are defined based on biomarker evidence and cognitive status, and they are intended for research purposes to identify individuals at risk for Alzheimer's disease and to facilitate early intervention studies.

Stage 1 with asymptomatic cerebral amyloidosis, is characterized by the presence of biomarker evidence of Aβ accumulation without any detectable signs of neurodegeneration or cognitive decline. Individuals in this stage show elevated tracer retention on PET amyloid imaging or low Aβ42 levels in cerebrospinal fluid (CSF). However, they do not exhibit markers of neuronal injury, such as elevated tau levels in CSF, hypometabolism on FDG-PET, or structural abnormalities on MRI. Additionally, there is no evidence of cognitive decline in these individuals.

Stage 2 is characterized by amyloid positivity with evidence of synaptic dysfunction and/or early Neurodegeneration. It involves individuals who not only have biomarker evidence of Aβ accumulation but also show signs of early neurodegeneration. These markers of neuronal injury include elevated CSF tau or phospho-tau, hypometabolism in regions typically affected by Alzheimer's disease (such as the posterior cingulate, precuneus, and temporoparietal cortices) on FDG-PET, and cortical thinning or hippocampal atrophy on structural MRI. Despite these changes, individuals in this stage do not exhibit cognitive decline.

Stage 3 is characterized by amyloid positivity with evidence of neurodegeneration and subtle cognitive decline. This stage represents individuals who have biomarker evidence of both amyloid accumulation and neurodegeneration, along with subtle cognitive decline that does not yet meet the criteria for mild cognitive impairment (MCI). These individuals show positive markers for Aβ accumulation and neuronal injury, and they also exhibit evidence of cognitive decline from their own baseline, even if their performance remains within the "normal" range on standardized cognitive tests.

The term "antigen-binding peptide", as used herein, refers to a peptide that specifically recognizes and binds to an antigen. These antigen-binding peptides can be engineered or naturally occurring and may be used in various applications, including therapeutic agents, diagnostic assays, and research tools to detect or neutralize specific antigens. The term comprises, antibodies, antibody fragments, Fab fragments, substantially intact antibodies, chimeric antibodies, bispecific antibodies, F(ab')2 fragments, and single chain Fv fragments.

The term "amount sufficient to induce an immune response", as used herein, refers to a quantity of an antigen or immunogen that is adequate to stimulate the body's immune system to recognize and respond to the antigen, typically resulting in the production of antibodies or activation of immune cells.

The terms "binds" and "binding", as used herein, preferably relate to specific binding. In some embodiments, the terms "binds" is to be understood as "is capable of binding", and "binding" is to be understood as "capable of binding"; accordingly, the term "is specific for" is to be understood as "is capable of specifically binding (to)", the term "specifically binds" is to be understood as "is capable of specifically binding (to)", and "specific binding" is to be understood as "capability of specific binding (to)".

The terms "is specific for", "specifically binds" and "specific binding", as used for example in the context of a molecule A being specific for a molecule B or a molecule A specifically binding to a molecule B or a molecule A showing specific binding for a molecule B, refer to a situation where molecule A binds to molecule B, but does not bind to other unrelated molecules, or with substantially reduced affinities. Such binding can be measured by routine methods, for example by competition ELISA or by measurement of affinity (K_{D}) by surface plasmon resonance measurements. Similarly, a molecule A being specific for an epitope C or a molecule A specifically binding to epitope C or a molecule A showing specific binding for epitope C, refer to a situation where molecule A binds to epitope C, but does not bind to other unrelated epitopes, or with substantially reduced affinities.

The term "treatment", as used herein, refers to the process of providing a subject with a pharmaceutical treatment, e.g., the administration of a drug, such that at least one symptom of the disease is decreased. Treatment of a disease can be improving the disease and/or curing the disease.

The term "prevention", as used herein, refers to the process of providing a subject with a pharmaceutical treatment, e.g., the administration of a drug, such that at least one symptom of the disease prevented from occurring.

The term "affinity tag", as used herein, refers to a moiety added to either the N- or C-terminus of a recombinant protein to enable or facilitate purification of the expressed protein. An affinity tag sequence usually contains from several to hundreds of amino acids.

The term "halo-tag", as used herein, refers to a protein tag derived from the bacterial enzyme haloalkane dehalogenase, designed to covalently bind to a synthetic ligand.

The term "ValCitPABC linker", as used herein, refers to a cleavable linker that consists of a Valine-Citrulline dipeptide and a PABC spacer. This linker can be cleaved by cathepsin B.

The term "GGFG tetrapeptidyl-aminomethoxy linker", as used herein, refers to a cleavable linker that combines tetrapeptide sequence GGFG with an aminomethoxy functional group. The GGFG sequence is recognized and cleaved by lysosomal proteases, such as cathepsins, which are enzymes found within the lysosomes of cells. The GGFG sequence ensures that the linker is cleaved specifically within the target cell's lysosomes. The aminomethoxy functional group acts as a spacer or a self-immolative moiety that facilitates the efficient release of the protein once the GGFG sequence is cleaved by the proteases.

The term "Azido-Phenyl-Amido-S-S-Sulfo-NHS linker", as used herein, refers to a bifunctional linker containing an azido group for click chemistry, a phenyl ring for stability, and a sulfo-NHS ester for efficient amine coupling, with a disulfide bond for cleavability.

The term "Azidoethyl-SS-propionic NHS ester linker", as used herein, refers to a bifunctional linker featuring an azido group for click chemistry, a disulfide bond for cleavability, and an NHS ester for amine-reactive conjugation.

The term "Biotin-PEG4-S-S-acid linker", as used herein, refers to a biotinylated linker with a polyethylene glycol (PEG4) spacer and a disulfide bond, terminating in a carboxylic acid group for further functionalization.

The term "Trifluoroacetamidoethyl-SS-propionic NHS ester linker", as used herein, refers to a bifunctional linker with a trifluoroacetamido group for stability, a disulfide bond for cleavability, and an NHS ester for amine-reactive conjugation.

The term "Aminoethyl-SS-ethylalcohol linker", as used herein, refers to a bifunctional linker containing an aminoethyl group for reactivity, a disulfide bond for cleavability, and an ethyl alcohol group for further functionalization.

The term "[a] nucleic acid", as used herein, refers to a biopolymer consisting of RNA or DNA that can be engineered into forms such as plasmids or vectors, as well as other delivery systems, all of which are suitable for transfection or transformation of a host cell to facilitate the introduction and expression of specific genetic material.

The term "immunopurification", as used herein, refers to a biochemical technique used to isolate a specific molecule, such as a protein, from a complex mixture using an antibody that specifically binds to that molecule. Said antibody that specifically recognizes and binds to the molecule, can be attached to a solid support, such as beads or a column matrix. The target molecule binds to the antibody, while other components of the mixture are washed away. The target molecule is then eluted (detached) from the antibody, usually by changing the conditions (such as pH or ionic strength) to disrupt the antibody-antigen interaction.

The term "suitable host cell", such as used for example in "suitable host cell for production of an ApoE", refers to a type of cell that is used to express and produce ApoE according to the present invention. These cells are chosen based on their ability to support the efficient production, proper folding, post-translational modification, and secretion of ApoE according to the present invention. For the present invention, the suitable host cell for the production of ApoE is preferably selected from a group comprising microorganisms, such as bacteria or yeasts; mammalian cells; human cells; animal cells; and animals; wherein, preferably, the suitable host cell is selected from bacteria, yeasts, and animal cells; more preferably, bacteria, and animal cells; even more preferably selected from an *Escherichia coli* strain, a HEK cell line, and a CHO cell line; wherein, most preferably, the suitable host cell is *E. coli* BL21.

The term "suitable animal host", as used herein, refers to an animal that is capable of expressing and producing a specific protein of interest. These animals are chosen based on their ability to produce the protein in a functional form and in sufficient quantities. For the present invention, the suitable animal host for a production of an antigen-binding peptide is preferably selected from the group comprising rodents, mice, rats, rabbits, chickens, sheep, guinea pigs, goats, donkey, horses and camelids; more preferably selected from mice, rats, and rabbits; even more preferably selected from mice and rats; even more preferably selected from wistar rats and CD1 mice.

The term "incomplete Freud's adjuvant", as used herein, refers to an oil-in-water emulsion used in immunology to enhance the immune response to an antigen. Incomplete Freud's adjuvant prolongs the presence of the antigen at the injection site, aiding in sustained immune stimulation, and is commonly used in booster immunizations and antibody production in research settings.

The term "keeping of animal" and the like, as used herein, refers to the practice of maintaining and caring for animals used in research or production, ensuring their well-being and proper conditions for experimental purposes.

The term "subcutaneous administration", as used herein, refers to the administration of a substance into the tissue layer between the skin and the muscle.

The term "intraperitoneal administration", as used herein, refers to the administration of a substance into the peritoneal cavity, the space within the abdomen that houses the intestines, liver, and other organs.

The term "screening of the hybridoma cells", as used herein, refers to a process of identifying and selecting hybridoma cells that produce the desired monoclonal antibody from a pool of hybridomas.

The term "antibody", as used herein, is used interchangeably with the term "immunoglobulin", and refers to a glycoprotein comprising at least two heavy (H) chains and two light (L) chains inter-connected by disulfide bonds. The term also includes all recombinant forms of antibodies, e.g. antibodies expressed in prokaryotes, unglycosylated antibodies and derivatives as described below. There are five different types of heavy chains, which define antibody isotypes of different functional activity: IgM, IgD, IgG, IgA and IgE. Each heavy chain comprises a heavy chain variable region (abbreviated herein as V_{H} region) and a heavy chain constant region. Each light chain comprises a light chain variable region (abbreviated herein as V_{L} region) and a light chain constant region. The V_{H} and V_{L} regions can be further subdivided into regions of hypervariability, termed complementarity determining regions (CDR), interspersed with regions that are more conserved, termed framework regions (FR). The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The terms "antibody fragment", "fragment of an antibody", "binding fragment of an antibody", and the like, as used herein, refer to one or more portions of an antibody that retain the ability to specifically interact (e.g. by binding, steric hindrance, stabilizing spatial distribution) an antigen. Examples of binding fragments include, but are not limited to, a Fab fragment, a monovalent fragment consisting of the V_{L}, V_{H}, CL and CH1 domains; a F(ab)2 fragment, a bivalent fragment comprising two Fab fragments linked by a disulfide bridge at the hinge region; a Fd fragment consisting of the V_{H} and CHI domains; a Fv fragment consisting of the V_{L} and V_{H} domains of a single arm of an antibody; a dAb fragment, which consists of a V_{H} domain; and an isolated complementarity determining region (CDR). Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv)). Such single chain antibodies are also intended to be encompassed within the term "fragment of an antibody", "binding fragment of an antibody", "antibody fragment" and the like.

The term "humanized antibody", as used herein, refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "human antibody", as used herein, refers to an antibody which comprises only sequences derived from human immunoglobulin sequences.

The term "chimeric-human antibody", as used herein, refers to an antibody composed of variable regions from a non-human species (e.g., mouse) and constant regions from a human antibody, combining elements from both sources.

The term "intact antibody", as used herein, refers to a complete antibody molecule with both heavy and light chains, including all functional regions (variable and constant regions).

The term "substantially intact antibody", as used herein, refers to an antibody that retains most of its structural and functional components, including the antigen-binding sites and Fc region, but may have minor modifications.

The term "Fab fragment", as used herein, refers to a fragment of an antibody that includes the antigen-binding sites (variable regions of one heavy and one light chain) but lacks the Fc region.

The term "F(ab')₂ fragment", as used herein, refers to a fragment of an antibody that consists of two Fab fragments linked by disulfide bonds, retaining the ability to bind antigens but lacking the Fc region.

The term "single chain Fv fragment", as used herein, refers to a fusion protein of the variable regions of the heavy and light chains of an antibody, connected by a short linker peptide, forming a single chain that can bind to antigens.

The term "immunized animal", as used herein, refers to an animal that has been intentionally exposed to a specific antigen to elicit an immune response that leads to the production of antigen-binding peptides, such as antibodies, against that antigen. These antigen-binding peptides, such as antibodies, can then be harvested from the animals' blood or other tissues.

The term "pharmaceutically acceptable carrier, diluent and/or excipient" refers to a non-toxic, inert, solid, semi-solid, or liquid diluent material or formulation auxiliary of any type.

"Pharmaceutically acceptable" in this context is meant to designate that said carrier is compatible with the other ingredients of the pharmaceutical composition and not harmful to the patient that the pharmaceutical composition is administered to. Examples of pharmaceutically acceptable carriers include, but are not limited to, water, water-propylene glycol solutions, or aqueous polyethylene glycol solutions.

The term "fusion proteins", as used herein, refers to chimeric proteins that are created through the joining of two or more genes that originally coded for separate proteins.

The term "administration", as used herein, refers to the application of a compound or a pharmaceutical composition thereof to a patient.

In one preferred embodiment, the antigen-binding peptide is an antibody that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 1.

In one preferred embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 2.

In one preferred embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 3.

In one preferred embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 4.

In one preferred embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 5.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 6.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 11.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 7.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 8.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 9.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 10.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 12.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 13.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No. 14.

In one embodiment, the antigen-binding peptide is an antibody or antibody fragment that specifically binds to fibrillary ApoE, wherein the ApoE comprises or consists of an amino acid sequence which is at least 90% identical, more preferably at least 95% identical, even more preferably at least 98% identical, even more preferably at least 99% identical, optionally identical, to an amino acid of SEQ ID No 15.

### BRIEF DESCRIPTION OF THE FIGURES

The present invention is now further described by reference to the following figures.

All methods mentioned in the figure descriptions below were carried out as described in detail in the examples.

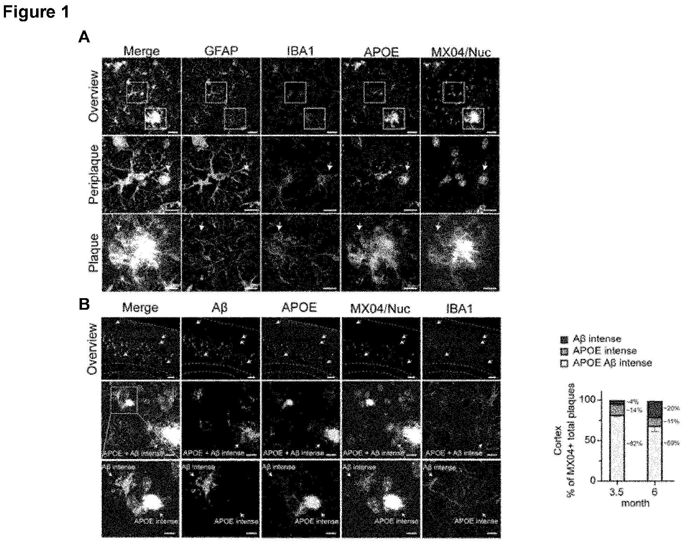

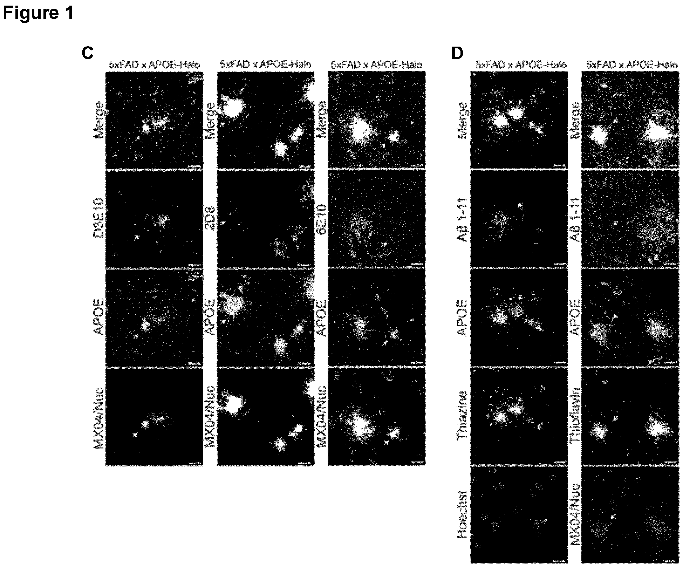

**Figure 1****: Detection of APOE aggregation in an amyloid mouse model by Halo Tag self-labeling technology**
   A) Representative immunostaining of APOE (visualized by Halo tag labeling) in GFAP+, IBA1+ cells and in MX04+ plaques in periplaque and plaque area. White arrows indicate IBA1+ cells expressing APOE (scale bar, top panel, 200µm; middle and bottom panel, 10 µm).
   B) Representative immunostaining of Aβ, APOE and MX04 positive plaques in 6-month-old 5xFAD ApoE-Halo mice. White arrows indicate APOE-intense plaques (scale bar, top panel, 200 µm; middle panel, 10 µm; bottom panel, 5 µm). Quantification of Aβ-intense, APOE-intense and Aβ/APOE-intense plaques in 3.5- and 6-months-old 5xFAD ApoE-Halo mice. Data are shown as mean percentage ± SEM (n=3 mice).
   C) Representative images showing APOE+ MX04+ plaques (white arrows) almost undetectable with antibodies (D3E10, 2D8 and 6E10) against Aβ in 5xFAD ApoE-Halo mice (Scale 10 µm).
   D) Representative images showing APOE+ aggregates in 5xFAD ApoE-Halo mice, undetectable with Aβ antibody 1-11, but recognizable with different β-sheet dyes (Thiazine, Thioflavin and MX04) (scale bar, 10 µm).

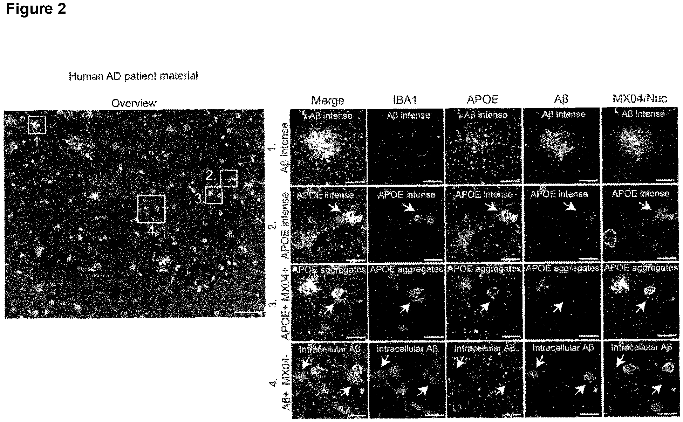

**Figure 2****: APOE aggregates in human AD autopsies**
   Left panel, overview scan of human AD brain section with MX04, Hoechst, APOE, Aβ, and IBA1 staining (scale bar, 50µm). Areas enclosed with white rectangles (1 to 4) are highlighted on the bottom. Right panel, representative images showing (1) extracellular Aβ intense plaque with fibrillar morphology (scale bar, 10 µm); (2) extracellular APOE intense plaque with compacted morphology (white arrows; scale bar, 10 µm); (3), intracellular APOE+ MX04+ aggregates in IBA1+ cells (white arrows; scale bar, 10 µm); (4) intracellular Aβ with no detectable APOE or MX04 staining in IBA1+ cells (white arrows; scale 10 µm).

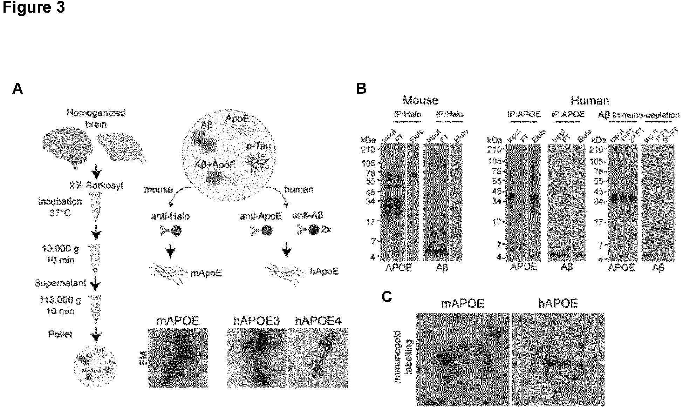

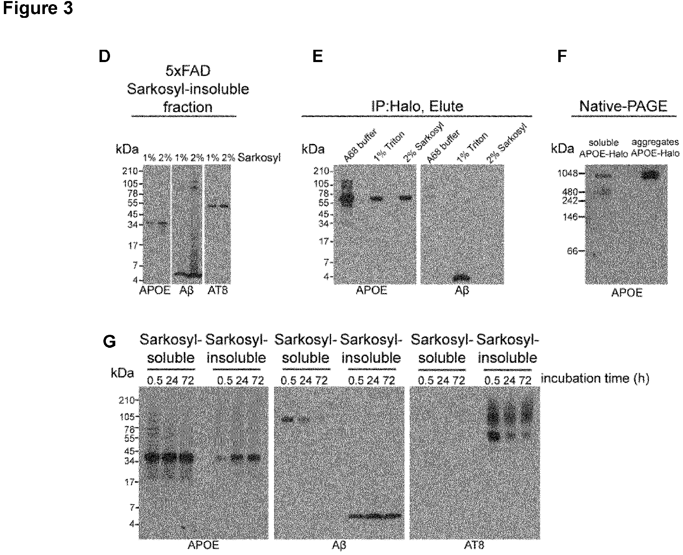

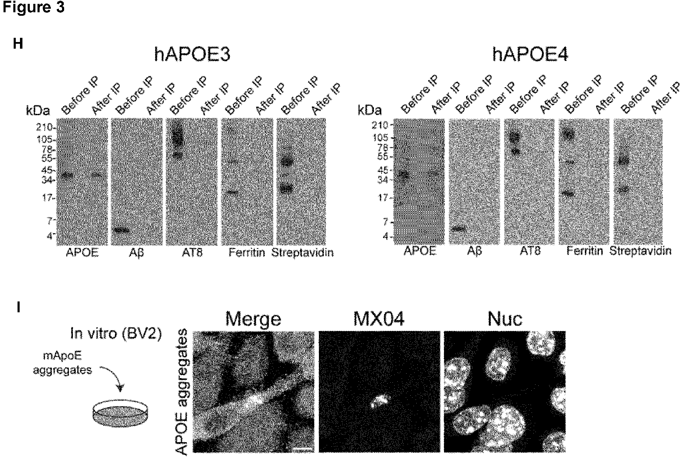

**Figure 3****: Isolation of APOE aggregates from 5xFAD mice and human AD autopsies**
   A) Schematic overview of mouse APOE (mAPOE) aggregate and human APOE (hAPOE) aggregate purification from 5xFAD ApoE-Halo mice and human AD patient material. Representative images show purified aggregates by electron microscopy (scale bar, 100 nm).
   B) Immunoblot analysis of purified mouse APOE aggregates (left) by Halo-immunoprecipitation, and human APOE aggregates (right) by APOE-immunoprecipitation combined with Aβ-immunodepletion.
   C) Immunoelectron microscopy of mouse APOE-Halo (mAPOE) aggregates and human APOE (hAPOE) aggregates. Anti-Halo and anti-human APOE antibodies were used for the immuno-gold labeling of mAPOE and hAPOE aggregates, respectively. White arrows highlight gold particles (scale bar, 50 nm).
   D) Immunoblot analysis of APOE, Aβ and Tau in sarkosyl-insoluble fractions, obtained by 1% and 2% sarkosyl extractions (30 min at 37°C) of 6-month-old 5xFAD mouse cortex showing the enrichment of insoluble ApoE from 2% sarkosyl. Samples were loaded to Tris-Tricine SDS gels (10-20%) with equal amount of total protein.
   E) Immunoblot analysis showing immuno-purified APOE-Halo obtained from A68 buffer-insoluble, 1% triton-insoluble and 2% sarkosyl-insoluble fractions from 3.5-month-old 5xFAD ApoE-Halo mice. Cortical tissue samples were extracted with either A68 buffer without detergent, with 1% triton, or with 2% sarkosyl. Brains tissue in the different lysis buffer were incubated for 30 min at 37°C. After immunoprecipitation by Halo-trap, elute from each fraction was loaded to Tris-Tricine SDS gels (10-20%) and analyzed for APOE and Aβ.
   F) Native PAGE analysis of soluble APOE-Halo (from 2-month-old ApoE-Halo mouse) and APOE-Halo aggregates (from 6-month-old 5xFAD ApoE-Halo mouse) obtained from cortical samples were separated on 12% Tris-Glycine native gel and analyzed by immunoblotting.
   G) Immunoblot analysis of APOE, Aβ and p-Tau (phosphorylated Tau) in 2% sarkosyl-insoluble fractions (extracted for 30 min, 24 h, and 72 h) of human AD tissue (temporal cortex). Brain tissues were extracted with 2% sarkosyl for 30 min, 24h or 72h at 37°C.
   H) Immunoblot analysis of APOE, Aβ, p-Tau, ferritin heavy chain, and streptavidin in sarkosyl-insoluble fraction (input, before IP) and immunopurified human APOE aggregates (elute, after IP) from human AD brains (APOE3/3 case and APOE4/4 case).
   I) Representative immunostaining shows MX04 positivity of immunopurified mouse APOE aggregates added to BV2 cells (scale bar, 5 µm).

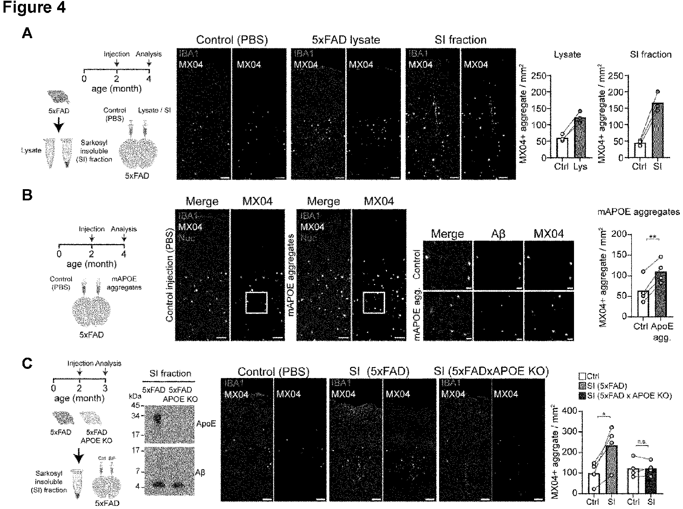

**Figure 4****: APOE aggregates act as a co-factor in β-amyloid pathology initiation**
   (A) PBS-extracted lysate (5xFAD lysate) and sarkosyl-insoluble (SI) fractions were isolated from 12-months-old 5xFAD mice, injected into the prefrontal cortex of 2-month-old 5xFAD mice, and compared to the PBS-control injected contralateral site (Control) to determine MX04+ plaque seeding. Tile scan images show the injection area with MX04 labelling for plaque visualization and IBA1+ cells (scale bar, 100 µm) with corresponding quantification of MX04+ plaques (lines connecting the data points indicate the comparison to PBS-control injected contralateral site).
   (B) Injection of immunopurified APOE aggregates into the prefrontal cortex of 2-month-old 5xFAD mice as indicated in the graphical illustration. Tile scan images show the injection area with MX04, IBA1 and Hoechst (Nuc) staining (scale 100 µm) two months after injection. Magnified images of white rectangles, of the injected brain area with MX04, Aβand IBA1 staining (scale 25 µm). Right, quantification of MX04+ aggregates in injected brain areas. Data points represent mean of 4 mice, **p < 0.01, Student's t-test, lines connecting the data points indicate the comparison to PBS-control injected contralateral site.
   (C) Injection of sarkosyl-insoluble (SI) fraction isolated from 5xFAD (12-month-old) or 5xFAD ApoE KO (12-month-old) mice into the prefrontal cortex of 2-month-old 5xFAD mice as indicated in the graphical illustration. Immunoblot analysis of APOE and Aβ concentration of injection material showing equal Aβ amount. Tile scan images show the injection area with MX04 labelling for plaque visualization and IBA1+ cells (scale 100 µm) with corresponding quantification of MX04+ plaques on the right. Data points represent mean of 4 mice, **p < 0.01, Student's t-test, lines connecting the data points indicate the comparison to PBS-control injected contralateral site.

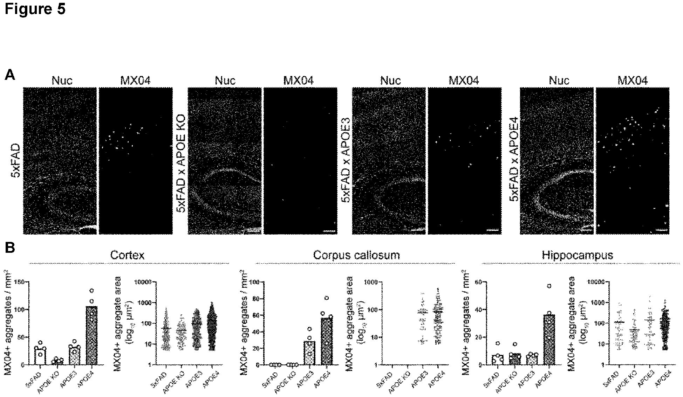

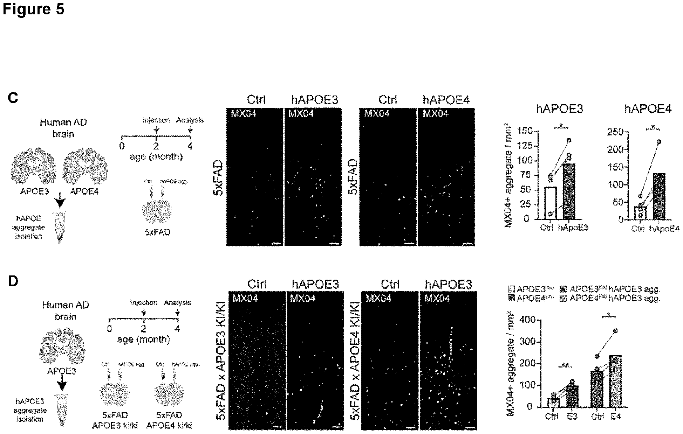

**Figure 5****: Human APOE aggregates induces β-amyloid pathology in** 5xFAD
   (A) Tile scan images show cortex and hippocampus of 3-month-old 5xFAD, 5xFAD APOE KO, 5xFAD APOE3 KI, and 5xFAD APOE4 KI mice with MX04 staining (scale 50 µm).
   (B) Quantification of MX04+ aggregates and violin plot showing the numbers and size distribution of MX04+ aggregates in different brain areas. Data points represent mean of 4 mice, *p < 0.05, ***p < 0.001, one-way ANOVA with Tukey's post hoc test.
   (C) Injection of immunopurified APOE aggregates from human AD brains (APOE3/3 and APOE4/4 patient-derived) into the prefrontal cortex of 2-month-old 5xFAD mice as indicated in the graphical illustration. Tile scan images show the injection area with MX04 (scale 100 µm) two months after injection. Right, quantification of MX04+ aggregates in injected brain areas. Data points represent mean of 4 mice, *p < 0.05, Student's t-test, lines connecting the data points indicate the comparison to PBS-control injected contralateral site.
   (D) Injection of immunopurified APOE aggregates from human AD brain (APOE3/3 patient-derived) into the prefrontal cortex of 2-month-old APOE3 KI 5xFAD and APOE4 KI 5xFAD mice as indicated in the graphical illustration. Tile scan images show the injection area with MX04 staining (scale 100 µm) two months after injection. Right, quantification of MX04+ aggregates in injected brain areas. Data points represent mean of 4 mice, *p < 0.05, **p < 0.01, Student's t-test, lines connecting the data points indicate the comparison to PBS-control injected contralateral site.

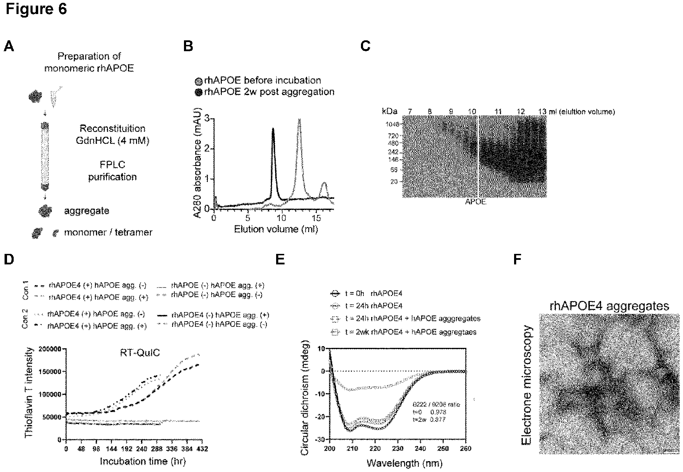

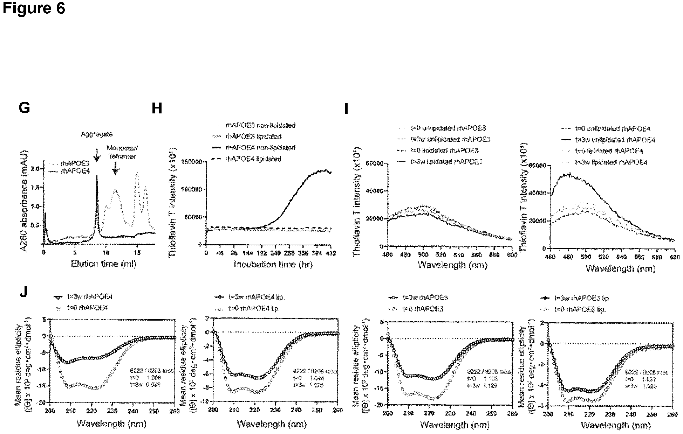

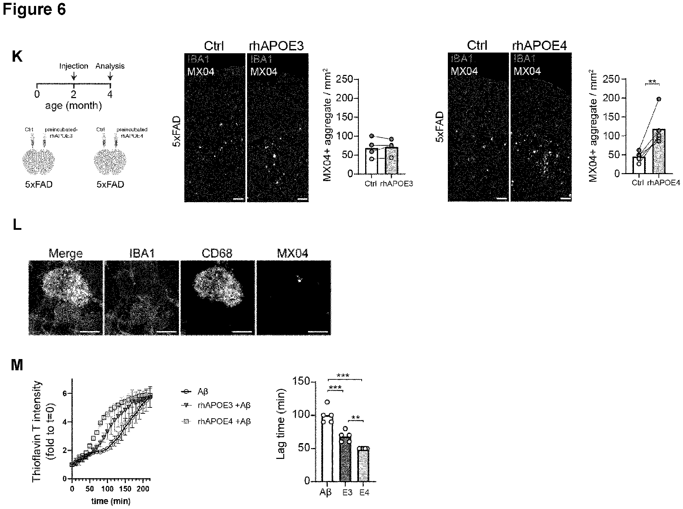

**Figure 6****: In vitro generated APOE4 aggregates induces β-amyloid pathology in 5xFAD**
   (A) Experimental scheme for the preparation of monomeric / tetrameric recombinant human APOE (rhAPOE) Monomeric / tetrameric rhAPOE was prepared via FPLC form recombinant APOE4 after unfolding by 4 mM guanidine hydrochloride (GdnHCL).
   (B) Monomeric / tetrameric rhAPOE was incubated at 37°C for up to 2 weeks. FPLC analysis shows the shift of elution volume peak before and after incubation (black: purified monomeric / tetrameric rhAPOE before incubation; grey: rhAPOE (0.5 mg/ml) incubated for 2 weeks at 37°C).
   (C) Native-PAGE analysis of FPLC fractionated recombinant human APOE4. Fractions corresponding to elution volume of 7-13 ml were separated by 4-12% Tris/Glycine native gel and analyzed by immunoblotting using anti-APOE antibody.
   (D) Chronological amplification of β-sheet structure in different preparations of rhAPOE by RT-QuIC analysis. Con.1: rhAPOE (0.22 mg/ml) + 10 µM Thioflavin T (240 µl of total volume) with and without human APOE aggregate from AD patient material, respectively. Con.2: rhAPOE (0.5 mg/ml) + 10 µM Thioflavin T (100 µl of total volume) with and without human APOE aggregate from AD patient material, respectively. Lines without ThT increase represent control buffer + 10 µM Thioflavin T in 100 µl and 240 µl of total volume, respectively (dashed line: without hAPOE aggregates, filled line with hAPOE aggregates).
   (E) Circular dichroism (CD) measurement before and after incubation (24 h and 2 weeks) of monomeric / tetrameric rhAPOE4 (0.15 mg/ml) in the presence or absence of isolated human APOE aggregates. Each value represents the average of 4 repeated measurements.
   (F) Representative EM image shows the morphology of rhAPOE after 2-week incubation at 37°C (scale bar, 100 nm).
   (G) Monomeric / tetrameric rhAPOE3 and rhAPOE4 (0.5 mg/ml) were incubated at 37°C for 4 weeks. FPLC analysis shows the difference of elution volume peaks between rhAPOE3 and rhAPOE4 after incubation (grey: rhAPOE3, black: rhAPOE4).
   (H) Chronological amplification of β-sheet structure in rhAPOE3 unlipidated and lipidated (grey) and rhAPOE4 unlipidated and lipidated (black) by RT-QuIC analysis.
   (I) Thioflavin T spectrometry of unlipidated (= monomeric / tetrameric) / lipidated rhAPOE4 and rhAPOE3 (14.2 µM) before and after 3-week incubation.
   (J) Circular dichroism (CD) measurement before and after 3-week incubation of unlipidated (= monomeric / tetrameric) / lipidated rhAPOE4 (0.5 mg/ml). Each value represents the average of 4 repeated measurements.
   (K) Injection of 3-week-preincubated rhAPOE3 and 3-week-preincubated rhAPOE4 (rhAPOE4 aggregates) into the prefrontal cortex of 2-month-old female 5xFAD as indicated in the graphical illustration. Tile scan images show the injection area with MX04 (gray) and IBA1 (magenta) staining (scale 100 µm) two months after injection with the corresponding quantification of MX04+ aggregates in injected brain areas on the right. Data points represent individual mice (n=4), *p < 0.05, Student's t-test, lines connecting the data points indicate the comparison to PBS-control injected contralateral site.
   (L) Injection of rhAPOE4 aggregates into the prefrontal cortex of 9-month-old wild-type mouse as indicated in the graphical illustration. Magnified view of aggregate injection area shows, IBA1 ,CD68, MX04 and nuclear staining 2 dyas after injections (scale bar, 10 µm).
   (M) Chronological amplification of β-sheet structure in different preparations of monomeric Aβ42 by RT-QuIC analysis. HFIP-treated Aβ42 (10 µM) was incubated either with 3-week-preincubated rhAPOE3 or rhAPOE4 (1 µg each) dissolved in TBS, or equal amount of TBS as control. Data points represent mean value ± SEM of 5 analysis. Bar graph represents mean rag time of each sample to reach 2-fold increase from baseline, **p < 0.01, ***p < 0.001, one-way ANOVA with Tukey's post hoc test.

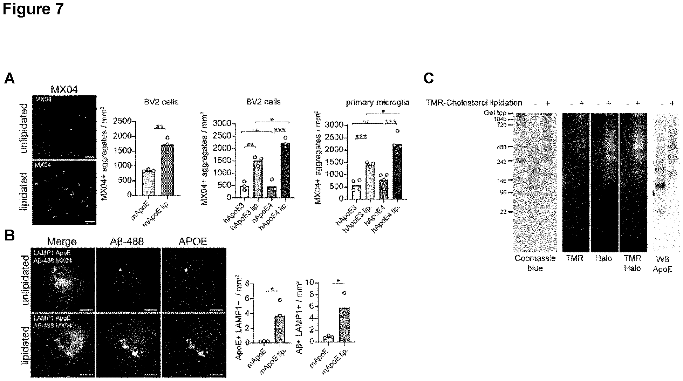

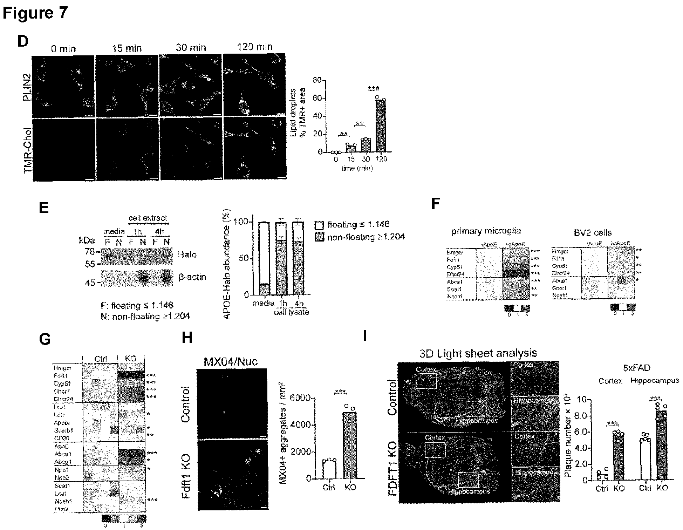

**Figure 7****: Microglia specific cholesterol synthesis ablation enhances MX04+ aggregate formation**
   (A) Representative images of MX04+ aggregates in BV2 cells 72 h after treatment with either POPC/Cholesterol-lipidated APOE + Aβ-42 or unlipidated APOE + Aβ-42 (scale bar, 20 µm) with corresponding quantification on the right. Middle: Quantification of MX04+ aggregate in BV2 cells, incubated for 72 h with Aβ and human APOE3 and APOE4 (unlipidated and lipidated). Right: Primary microglia cells 72 h after treatment with either POPC/cholesterol-lipidated hAPOE3 or hAPOE4 (0.1 µg/ml) + Aβ-42 (1 µg/ml) or unlipidated hAPOE3 or hAPOE4 (0.1 µg/ml) + Aβ-42 (1 µg/ml). Bar graphs represent mean number of MX04+ aggregates, n=3-4 independent cultures, **p < 0.01, Student's t-test (BV2), *p < 0.05, **p < 0.01, ***p < 0.001, one-way ANOVA with Tukey's post hoc test.
   (B) Representative images of internalized Aβ-488 and APOE (His) in IBA1+ BV2 cells incubated for 4h with lipidated APOE + Aβ-42 or unlipidated APOE + Aβ-42 (Scale 10 µm). Uptake of N-terminally His-tagged recombinant mouse APOE was evaluated by His-tag staining. Data represent mean number of MX04+ aggregates, n=3 independent cultures, *p < 0.05, Student's t-test.
   (C) Coomassie blue staining/fluorescence analysis of Native-PAGE gel and immunoblot analysis of TMR-cholesterol lipidated APOE-Halo and unlipidated APOE-Halo. Left lane in Coomassie blue staining represents protein marker.
   (D) Representative images of TMR-cholesterol+ PLIN2+ lipid droplets in primary microglia cells after 0, 15, 30, and 120 minutes treatment with TMR-cholesterol lipidated APOE-Halo (0.1 µg/ml) with corresponding quantifications. Bar graphs represent % of TMR-cholesterol+ area in PLIN2+ lipid droplets, n = 3 independent cultures, **p < 0.01, ***p < 0.001, one-way ANOVA with Tukey's post hoc test.
   (E) Extraction protocol for floating fractions (lipidated APOE) and non-floating fractions (unlipidated APOE) from microglia treated with lipidated APOE-Halo (0.2 µM) using Optiprep density ultracentrifugation. Representative immunoblot of media and cell extracts after 1 and 4 hours with corresponding quantifications on the right. Bar graphs represent APOE-Halo abundance in floating and non-floating fractions of media and cell extracts.
   (F) qPCR analysis of transcripts in cholesterol synthesis and transport in primary microglia and BV2 cells 24 h after treatment with lipidated APOE + Aβ-42 or unlipidated APOE + Aβ-42. Heatmaps represent individual expression levels as fold to unlipidated condition, n=3 cultures, *p < 0.05, **p < 0.01, ***p < 0.001, one-way ANOVA with Tukey's post hoc test.
   (G) qPCR analysis of transcripts in cholesterol synthesis, metabolism and transport in control BV2 and Fdft1-deficient BV2 cells. Heatmaps represent individual expression level as fold to control, n=3-4 independent cultures, *p < 0.05, **p < 0.01, ***p < 0.001, Student's t-test.
   (H) Representative images of MX04+ aggregates in Fdft1-deficient and control BV2 cells 72 h after incubation with lipidated APOE + Aβ-42 (scale bar, 10µm). Data represent mean number of MX04+ aggregates, n=3 independent cultures, ***p < 0.001, Student's t-test.
   (I) Left panel, plaque analysis by 3D light sheet microscopy in CX3CR1CreERT2 FDFT1flox/flox 5xFAD (FDFT1 KO 5xFAD) and corresponding controls CX3CR1CreERT2 FDFT1wt/wt 5xFAD (CX3CR1Cre 5xFAD) mice at 3.5 months with corresponding quantification on the right. Representative images show Congo red+ plaques in the isocortex and hippocampus. Data represent mean number of plaques in cortex and hippocampus, n=5 mice, ***p < 0.001, Student's t-test.

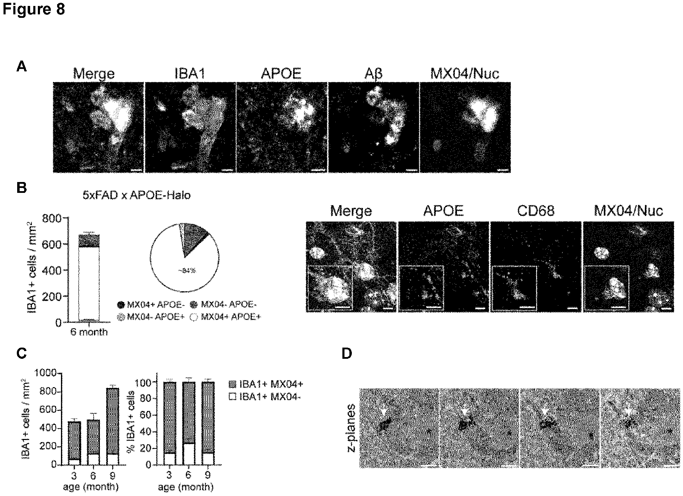

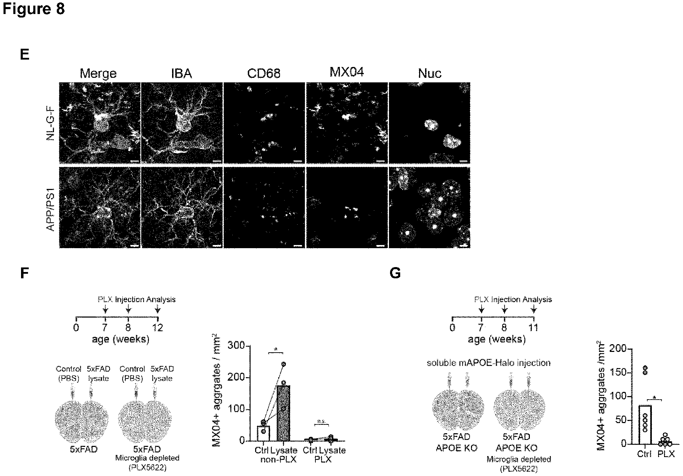

Figure 8: Microglial **β-amyloid** seeding requires APOE
   (A) Representative images of APOE+, Aβ+, MX04+ co-staining in IBA1+ cells) in human AD brain sections (scale bar, 10 µm).
   (B) Quantification of MX04+ aggregates colocalizing with APOE-Halo in 6-month-old 5xFAD ApoE-Halo mice. Bar graph data are shown as mean number of IBA1+ cells ± SEM. Pie chart shows the mean percentage of total IBA1+ cells positive or negative for MX04 and APOE-Halo (n=3 mice). Representative images (scale bar, 5 µm) of APOE-Halo+, MX04+ aggregates together with nuclear labeling and CD68+ IBA1+ cells of 6-months-old 5xFAD ApoE-Halo mice.
   (C) Quantification of MX04+CD68+ co-staining in IBA1+ cells of 5xFAD mice. Data represent mean numbers and percentages ± SEM of MX04+ IBA1+ and MX04- IBA1+ cells (n=3-4 mice).
   (D) Representative EM images of fibrillary lysosomal aggregates (white arrow) in microglia of 5xFAD mice (serial optical sections). Asterisk marks plaque core (scale bar, 10 µm).
   (E) Representative image of MX04+CD68+ co-staining in IBA1+ cells of APP/PS1 mice (12 month) and NL-G-F mice (12 month) (scale bar, 10 µm).
   (F) Left: Schematic overview of injection experiments following microglia depletion. Quantification of MX04+ aggregates in injected brain areas. Data points represent mean of 3 mice, *p < 0.05, **p < 0.01, Student's t-test, lines connecting the data points indicate the comparison to PBS-control injected contralateral site.
   (G) Left: Schematic overview of purified soluble APOE-Halo injection experiments in microglia-depleted 5xFAD ApoE-/- mice. Quantification of MX04+ aggregates. Data points represent mean of 3 mice, *p < 0.05, Student's t-test.

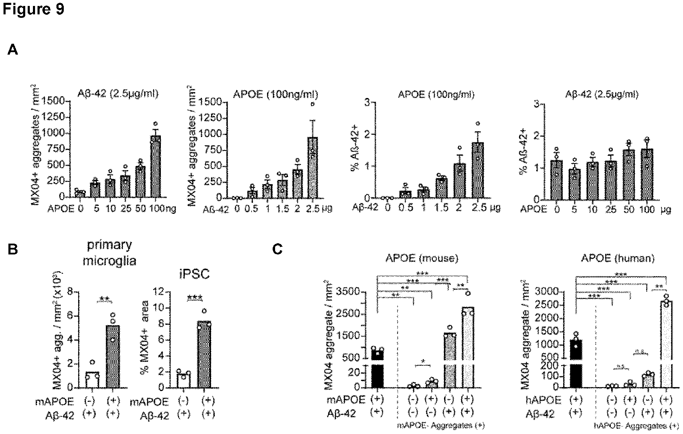

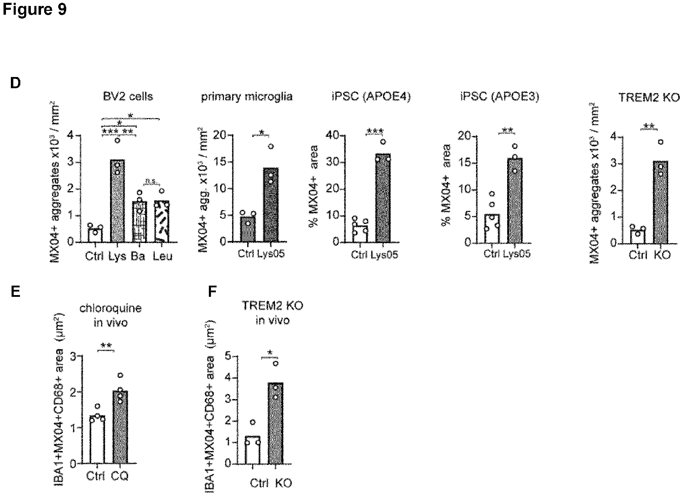

**Figure 9****: Lysosomal dysfunction promotes APOE-induced MX04+ aggregate formation in microglia**
   (A) Quantification of MX04+ aggregates with increasing Aβ-42 or APOE concentrations. Bar graphs represent mean number of MX04+ aggregates ± SEM of 3 individual cell cultures.
   (B) Quantification of MX04+ aggregate induction byAPOE/Aβ-42 co-treatment in primary microglia and iPSC microglia. Cells were incubated for 3 days with recombinant APOE (100 ng/ml) and/or Aβ-42 (2.5µg/ml). Bar graphs represent mean number of MX04+ aggregates of 3 individual cell cultures, **p < 0.01, ***p < 0.001, Student's t-test.
   (C) Quantification of intracellular MX04+ aggregates induced by mouse and human APOE aggregates in BV2 cells, incubated with immunopurified APOE aggregates in combination with recombinant APOE (100 ng/ml) and/or Aβ-42 (2.5µg/ml). Data points represent mean of 3 individual cultures, **p < 0.01, ***p < 0.001, one-way ANOVA with Tukey's post hoc test.
   (D) Quantification of MX04+ aggregate number in BV2 cells, primary microglia and iPSC derived microglia following lysosomal inhibitor treatment (Lys: Lys05; Ba: Bafilomycin; Leu: Leupeptin) and TREM2 KO BV2 cells. Data points represent mean of 3 individual cultures. BV2 cells *p < 0.05, **p < 0.01, ***p < 0.001, one-way ANOVA with Tukey's post hoc test. Primary microglia, iPSC, TREM2 KO *p < 0.05, **p < 0.01, Student's t-test.
   (E) Quantification of IBA1+/MX04+/CD68+ area following chloroquine administration in vivo. Chloroquine or equal amount of saline was injected to 5xFAD mice intraperitoneally every day for 7 days, and mice were sacrificed on the following day after the final injection. Data points represent mean of 4 mice, **p < 0.01, Student's t-test.
   (F) Quantification of IBA1+/MX04+/CD68+ area from APP/PS1 TREM2 KO mice compared to control (APP/PS1) (12 month). Data represent the mean area of MX04+ aggregates in CD68+IBA1+ cells (n=3 mice), *p < 0.05, Student's t-test.

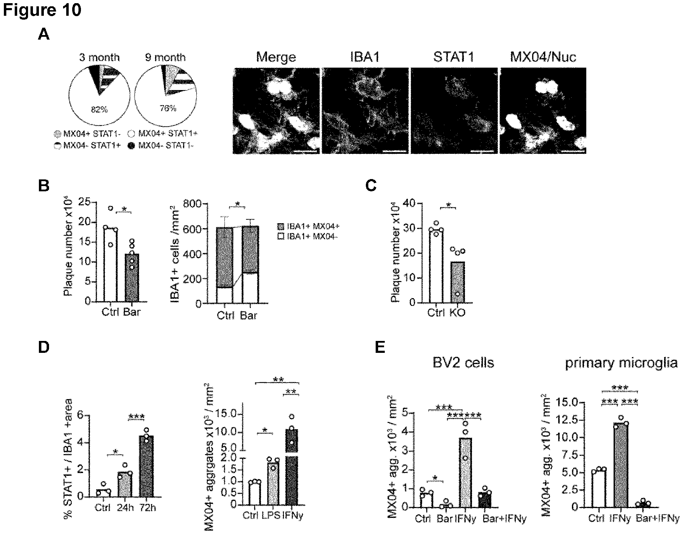

**Figure 10****: Microglial JAK/STAT1 inhibition reduces amyloid pathology**
   (A) Pie chart shows the mean percentage of total IBA1+ cells positive or negative for STAT1 and/or MX04 at 3 and 9 months of age (n=3-4 mice). Representative images of STAT1+MX04+ cells in 6-months-old 5xFAD mice (scare bar, 10 µm).
   (B) Left: Mean Congo red+ aggregate number in 5xFAD mice (4.5 months) treated with or without baricitinib (JAK/STAT inhibition). *p < 0.05, Student's t-test. Right: Quantification of MX04+ or MX04- IBA1+ cells in 5xFAD mice (4.5 months) treated with or without baricitinib (n=3 mice). Bar graph represent the mean of MX04+ or MX04- IBA1+ cells ± SEM. *p < 0.05, Student's t-test.
   (C) Mean Congo red+ aggregate number in 5xFAD and 5xFAD STAT1 KO mice at the age of 3.5 months. *p < 0.05, Student's t-test.
   (D) Left: Quantification for STAT1+ BV2 cells 24h and 72h after incubation with APOE (100 ng/ml) and Aβ-42 (2.5µg/ml). Data represent mean percentage of STAT1+ per IBA1+ area, n=3 individual cultures, *p <0.05, ***p< 0.001, one-way ANOVA with Tukey's post hoc test. Right: Quantification for MX04+ aggregates in BV2 cells, treated for 72h with either lipopolysaccharide (LPS) or IFNγ together with APOE/Aβ-42. Data represent mean number of MX04+ aggregates, n=3 independent cultures, *p < 0.05, **p < 0.01, one-way ANOVA with Tukey's post hoc test.
   (E) Quantification of MX04+ aggregates in BV2 cells and primary microglia, treated with IFNγ and/or Baricitinib following APOE (100 ng/ml) and Aβ-42 (2.5µg/ml) aggregate induction. Data represent mean number of MX04+ aggregates, n=3 independent cultures, ***p < 0.001, by one-way ANOVA with Tukey's post hoc test.

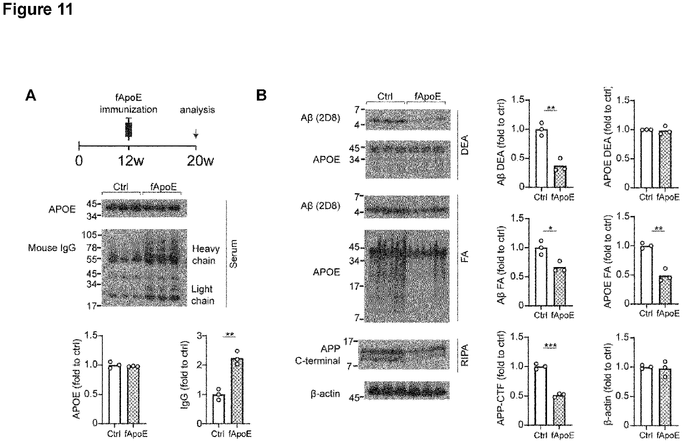

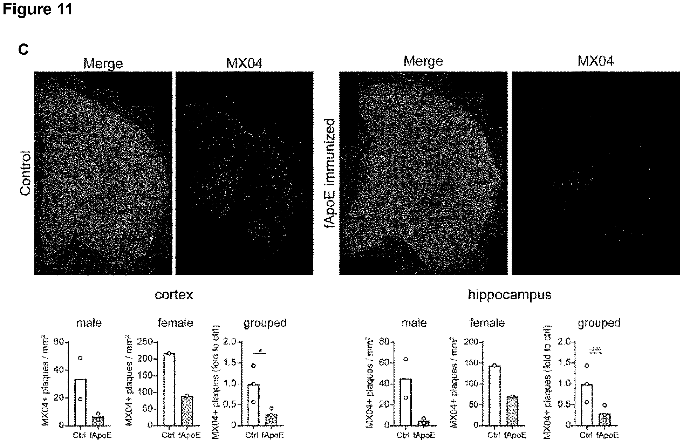

Figure **11****:** Active immunization against fibrillary **ApoE**4 diminishes **MX04+** plaques and reduces insoluble ApoE and **Aβ**
   (A) Schematic overview of the active immunization of 12-week-old 5xFAD x APOE4-KI mice with fibrillary recombinant ApoE4. Immunoblot analysis of serum shows serum ApoE levels and serum IgG amount divided in heavy and light chain 8 weeks post immunization. Bar graphs show fold to control changes of individual animals.
   (B) Immunoblot analysis of Aβ and ApoE in formic acid extracted (FA), diethylamine extracted fractions (DEA), and C-terminal fragments of amyloid precursor protein in RIPA extracted fractions from 5xFAD x APOE4 KI and 5xFAD x APOE4 KI immunized against fibrillary APOE4. Quantification showing fold to control changes of Aβ in FA/DEA, APOE in FA/DEA and APP in RIPA normalized by β-actin. Data points represent mean values.
   (C) Representative immunostaining of MX04 positive plaques in 20-week-old 5xFAD x APOE4KI mice and 5xFAD x APOE4KI mice immunized with fibrillary APOE4. Quantification of MX04+ plaques in male, female and grouped 5xFAD x APOE4KI mice showing plaques in cortex and hippocampus. Data are shown as fold to control ± SEM (n=1-3 mice).

In the following, reference is made to the examples, which are given to illustrate, not to limit the present invention.

### EXAMPLES

### Example 1

### EXPERIMENTAL MODEL AND SUBJECT DETAILS

### Human autopsy samples

Human AD patient material was obtained from the Neurobank Munich and experiments were performed with full ethical consent from the ethics committee at the LMU Munich.

### Cell lines and primary cells

Primary mouse microglia, astrocytes, and mixed glial cells were established from 2-15 days old *C57BL*/*6J* mice. Generation of *FDFT1* and *TREM2 KO* BV2 is described in METHOD DETAILS.

### Mice

All experiments were performed in accordance with the German animal welfare law and local regulations for animal experiments of Bavaria and Lower-Saxony. Animals were group housed (3-5 mice) in a controlled environment (temperature of 22°C, 30-70% humidity, 12 h dark/12 h light cycle) and had access to food and water ad libitum under specific pathogen free conditions. Male and female *C57BL*/*6J* mice (Age > 6 weeks if not state otherwise) or genetic mouse mutants (Age > 6 weeks if not state otherwise) were taken for all experiments. Animals of the same sex and age were randomly assigned to experimental groups (3-6 mice per group). Mice from different genotypes were cohoused. Mice did not undergo any procedures prior to the described experiments. Mice used in this study include *5xFAD, 5xFADApoE-Halo, 5xFAD ApoE KO, APP-NL-G-F, APP*/*PS1, APP*/*PS1 Trem2-KO, 5xFAD CR3CR1Cre-YFP, 5xFAD FDFT1 KO* mice.

### METHOD DETAILS

### Mice

*C57BL*/*6J, 5xFAD, ApoE KO, Cx3Cr1-CreERT2* mice were purchased from Charles River Laboratories. *APOE₃ KI, APOE₄ KI, Stat1 KO* mice were purchased from Jackson Laboratory. Generation of *ApoE-Halo* mice is described below. *5xFAD* mice were crossbred to *ApoE KO* to generate *5xFAD ApoE KO* mice (paternal *5xFAD* transgene delivery). *5xFAD ApoE-Halo* mice were generated by cross-breeding *5xFAD* mice with *ApoE-Halo* mice. *APOE3 KI* or *APOE4 KI* mice were crossbred with *5xFAD* mice (paternal *5xFAD* transgene delivery) to generate *5xFAD APOE3 KI* and *5xFAD APOE4 KI* mice. *STAT1 KO* mice were crossbred with *5xFAD* mice to generate *STAT1 KO 5xFAD* mice (paternal *5xFAD* transgene delivery). Conditional cholesterol synthesis mutants were generated by cross-breeding *Cx3Cr1-CreERT₂* mice with mice harboring *SQS*/*FDFT1* with two *loxP* sites flanking exon 5, referred to as *FDFT1 KO (CX3CR1 CreERT2* +/- *FDFT1*^{*flox*/}*^{flox}).* Conditional mouse mutants and respective controls received tamoxifen dissolved in corn oil by intraperitoneal injections in five consecutive days at a concentration of 75 µg/g of body weight to induce recombination. Conditional mutants (*FDFT1 KO 5xFAD, CX3CR1CreERT2* +/- *FDFT1*^{*flox*/}*^{flox} 5xFAD* +/-) were compared to the respective controls (*Cx3Cr1Cre 5xFAD; CX3CR1CreERT2* +/- *5xFAD* +/-).

### Generation of APOE-Halo reporter mice

APOE-Halo reporter mice were generated by CRISPR/Cas9-assisted gene editing in mouse zygotes. Briefly, pronuclear stage zygotes were obtained by mating *C57BL*/*6J* males with superovulated *C57BL*/*6J* females (Charles River). Embryos were then microinjected into the male pronucleus with an *ApoE*-specific CRISPR/Cas9 ribonucleoprotein (RNP) solution consisting of 30 ng/µl SpCas9 HiFi protein (IDT), 0.6 µM crRNA (IDT), 0.6 µM tracrRNA (IDT), and 5 ng/µl of a mutagenic targeting vector for the introduction of a HaloTag linker and the HaloTag7 coding sequence (Promega) right before the *ApoE* stop codon. The lengths of the homology arms were 900 and 1000 bp, respectively. After microinjection, treated zygotes were transferred into pseudopregnant CD-1 foster animals. Mutant founder animals were crossed to *C57BL*/*6J* animals to isolate the respective allele. Validation of the targeted loci was performed on genomic DNA from F1 animals by RFLP, Sanger sequencing, and qPCR-based copy number assays. To exclude additional unwanted modifications, putative off-target sites of the *ApoE*-specific crRNA were predicted using the CRISPOR online tool. Predicted loci were PCR-amplified and verified by Sanger sequencing. Validated animals without any off-target mutations were used for further breeding. All animal were housed in standard cages in a specific pathogen-free facility on a 12 h light/dark cycle with ad libitum access to food and water.

### Treatments to mice

Mice were fed normal chow (ssniff V1124), if not specified. For the cohort of microglial depletion, *5xFAD* and *5xFAD ApoE KO* mice were fed either Control AIN-76A standard chow or 0.12% PLX5622 (MedChemExpress, formulated in AIN-76A standard chow by Research Diets Inc.). Treatments were started at the age of 7 weeks provided ad libitum and continued until the age of 11-12 weeks. For the cohort of Baricitinib treatment, *5xFAD* mice were fed powdered chow (ssniff V1120) or powdered chow supplemented with Baricitinib (InvivoChem, V0338,) 10 mg/kg body weight solved in corn oil. Treatments were started at the age of 6 weeks provided ad libitum. For the chloroquine treatment, *5xFAD* mice were injected intraperitoneally every day with 45 mg/kg bodyweight (Sigma) while control mice were injected with the same amount of saline. Treatments were started at the age of 11 weeks for 7 days.

### Isolation of serum and brain APOE-Halo from ApoE-Halo mice for EM analysis

Isolation of APOE-Halo positive lipoparticles from serum and brain lysate was performed by immunoprecipitation using the ChromoTek Halo-Trap Magnetic Agarose Kit (Proteintech) according to manufacturer's instruction. Serum from *ApoE-Halo* animals was collected as described above, and 200 µl of serum was used as the input material for immunoprecipitation. For the preparation of brain-derived HDL-like lipoparticles, brain tissue was homogenized with RIPA buffer (10 mM Tris pH 7.5, 150 mM NaCl, 0.5 mM EDTA, 0.1% SDS, 1% Triton^{™} X-100,1% deoxycholate) followed by centrifuge at 17,000 g for 10 min at 4°C. The supernatant was subjected to a density gradient using OptiPrep^{™} Density Gradient Medium (Sigma) to separate the HDL-like lipoparticles. The fraction corresponding to the density of 1.146-1.204 g/ml was collected as a lipoparticle fraction after the ultracentrifugation at 54.000 g for 3 h at 4°C, and 200 µl of purified lipoparticles was used as the input material for immunoprecipitation. Each sample was analyzed by EM immediately after the isolation.

### mRNA expression analysis

For expression analysis of cell lysate, RNA was extracted using RNeasy MikroKit (Qiagen). RNA concentration and quality of cell culture samples were determined using NanoDrop spectrometer, and cDNA was synthesized with Superscript III (Invitrogen). Sorted cell populations were further processed for RNA/cDNA amplification according to Ovation PicoSL WTA system v₂ protocol (NuGEN). Quantitative PCRs were done in triplicates using the GoTaq qPCR master mix (Promega) or Powerup Sybr Green master mix (Applied biosystems) and the LightCycler 480 (Roche Diagnostics). Measured Ct values were normalized to the mean of housekeeping genes (*18S, Hprt, Rps13, Rplpo).* Quantification was done by applying the ΔΔCt method, normalized to experimental controls. All primers were designed to fulfill optimal criteria e.g. length, melting temperature, GC content, low number of repeats, and amplicon length. All primers were intron-spanning.

### Generation of BV2 FDFT1 and TREM2 knockouts

In order to generate the *FDFT1 KO* in BV₂ cells, we used Cas9-gRNA Ribonuclease complexes to generate a cut in the genome 5' and 3' of *Fdft1* Exon 3. The online tool CRISPOR (https://doi.org/10.1093/nar/gky354) was used to select suitable Guide RNAs, with following settings. Genome: "mus musculus UCSC Jun 2020 GRCm39/mm39"; Protospacer Adjacent Motif (PAM): '20bp-NGG - Cas9 S. Pyogenes'. The Guide RNAs used to generate the Knock Out were ATGGCGTGCCTGTAAAAAGA (including 5' addition of G) and GAGGACTTCCCCACGGTACT for the 5' and 3' cut, respectively. The RNP complexes for each guide RNA were generated individually at a ratio of 52 pmol:60 pmol (Cas9:gRNA) and co-nucleofected into BV2 cells using the P3 Primary Cell 4D-Nucleofector^{™} X Kit S. After 3 days, single cell clones were picked to ensure clonality of the generated population. Knockout of Exon 3 was confirmed via PCR using *Fdft1*Exon3KO-genotyping primers flanking the cut sites. Sanger Sequencing of the genotyping PCR product, and qPCR after serum starvation. All Guides were synthesized by Synthego and purchased as CRISPRevolution sgRNA EZ Kit. To generate the *TREM 2 KO* in BV2 cells the pSpCas9(BB)-2A-GFP plasmid (PX458; targeting Exon 2 with the gRNA CATGGCCGGCCAGTCCTTG) was used. 24 hours after nucleofection GFP-positive cells were isolated by FACS. Single-cell clones from the GFP-positive fraction were obtained by serial dilution and screened for genetic modifications in *Trem2* via PCR amplification of exons 2 and missense detection using T7 endonuclease I (NEB). Media and lysates of edited clones were analyzed by Western blot for TREM2 expression.

### Generation of iPSC derived microglia cells

All stem cell work was performed following all relevant guidelines and regulations. Female human episomal iPSC line A18944 was obtained from ThermoFisher (#A18945). Human iPSC-derived hematopoietic progenitors were generated by using Stem Cell Technologies STEMdiff^{™} Hematopoietic Kit (# 05310) according to the manufacturer's directions. Briefly, iPSCs were split once they were 70-90% confluent after 5 min incubation in PBS/ EDTA in 1:100 ration into GelTrex coated 6-well plates to get around ~20 small colonies per well. Cells were left to recover overnight, and on the next day, the culture medium was changed to STEMdiff basal media containing supplement A. On day 2 days half of the medium was replaced with a fresh one. The next day (day 3) the medium was switched with STEMdiff basal media containing supplement B. Every other day (days 5, 7, 10) half of the medium was replaced with a fresh one. Cells were harvested on day 12 by collecting cell suspension, filtering through a 70µm cell strainer, and titrating with DMEM/F12 and differentiated into microglia cells. Therefore, cells were plated onto GelTrexcoated 6-well plates with 2 ml of iMGL-DM1 media with a cell density of 160.000-200.000 cells per well. iMGL-DM1 media contained the following growth factors: 25 ng/ml M-CSF (Peprotech, #300-25), 100 ng/ml IL34 (Peprotech, #200-34), and 50 ng/ml TGF-b (Peprotech, #AF-100-21C). Cells were fed with 1 ml of medium every other day. Cells were split every 6-8 days if they were too confluent. iPSC were maintained on VTN-coated 6-well cell culture plates in a humidified incubator (5% CO₂, 370C). Experiments were conducted after 16 days of microglia differentiation.

### Aggregation assay in cell culture

Aggregation assays were performed in BV2 macrophage mouse cell line and primary mouse glial cells. Wildtype BV2 cells and *Trem2* knockout cells and *FDFT1 KO* cells were plated (1 x 10⁴). Primary mouse microglia was established from 2-15 days old mice. Briefly, brains were digested using the neuronal tissue dissociation protocol (Miltenyi biotec), and cell solution was strained using a 40µm strainer to get a single cell solution. Cell solution was then incubated with CD11b microbeads followed by incubation, and microglia was positively selected using LS columns and plated (4 × 10⁴) on a PLL coated coverslips for imaging. Isolated primary microglia were cultivated in DMEM containing 1% penicillin-streptomycin, 1mM sodium pyruvate and 10 ng/ml M-CSF until confluent. For preparation of primary astrocytes, cell solution was incubated with ACSA-2 microbeads, plated (4 × 10⁵) on a PLL coated coverslips and cultivated in DMEM containing 1% penicillin-streptomycin and 20% fetal bovine serum. Preparation of mixed glial cell culture was performed. Cerebral cortices from P2 pups were dissected, minced, and digested. Dissociated cells were plated in poly-D-lysine-coated 75 cm² flasks, and maintained in DMEM containing 20% fetal bovine serum and 1% penicillin-streptomycin. After the cells became confluent (~10 days), cells were trypsinized and plated (4 × 10⁵) on a PLL coated coverslips and cultivated until use. Cultures were used for following treatments when reached 80% confluency and a ratio of about 80% astrocytes and 20% microglia cells.

If not stated differently, the following four aggregation treatment groups were prepared and incubated for 3 days until analysis. Namely, cells were treated either with a single dose of Aβ₄₂ (Anaspec, AS-64129-1; 1 µg/ml), a single dose of recombinant mouse APOE (N-terminally His-tagged, BiologicsCorp, 100 ng/ml), or a double treatment containing Aβ42 and APOE while control untreated cells were cultured in parallel. HFIP momoeric Aβ42 peptide was incubated in 1xPBS pH 7.4 at 37°C for 48 hours and directly applied to cell culture experiments. After 72 h of incubation, cells were washed and fixed with 4% paraformaldehyde and stored until staining. To test if purified APOE aggregates from *5xFAD* mice and human AD brain affects aggregation in BV2 cells, purified materials (100 ng/ml) were supplemented in addition to the above-mentioned standard treatments. Lysosomal inhibitors, Lys05 (1 µM), Bafilomycin (7,5 nM), and Leupeptin (5 µM), were added 1 h after the treatment of Aβ42 and APOE, and cells were collected after 72 h. For induction of microglial activation, BV2 cells and primary microglia were treated with LPS (10 ng/ml), IFNγ (1 ng/ml) and Baricitinib (1 µM; InvivoChem,V0338) 2-24 h before Aβ42 and APOE treatment. For comparing the effect of unlipidated and lipidated APOE, recombinant mouse APOE and POPC/cholesterol-lipidated mouse APOE (2.9 nM each) were used in single treatment or in combination with Aβ42. Cells were incubated for 72 h for the assessment of MX04⁺ aggregates, and for 4 h for the assessment of intracellular Aβ/APOE uptake. For analysis of Aβ and APOE concentration-dependency against aggregate formation, consistent concentrations of APOE (100 ng/ml) or Aβ (2.5 µg/ml) were applied in combination with varying concentrations of the other protein (Aβ: 0-2.5 µg/ml, APOE: 0-100 ng/ml).

### Preparation of amyloid protein extracts

For Western blotting of amyloid proteins, mouse brains were perfused with PBS, dissected into cortices and other regions, snap-frozen, and stored at -8o°C until use. After pulverization of cortices by a liquid nitrogen cooled mini mortar (Sigma), 20-25 mg of brain powder were transferred to soft tissue homogenizing Precellys tubes (Bertin Technologies). 130-150 µl of ice-cold DEA buffer (0.2% diethylamine in 50 mM NaCl, pH 10, and protease inhibitor mix (Life)) was added to generate soluble extracts. The powder was homogenized at 6,500 rpm. for 30 s at 4°C using a sample homogenizer (Bertin Technologies). The pellet from 10 min centrifuge at 4,000 g (4°C) was resuspended in 130-150 µl RIPA buffer (20 mM Tris/HCl pH 7.5, 150 mM NaCl, 2.5 mM EDTA, 1% NP-40, 1% sodium deoxycholate, 2.5 mM sodium pyrophosphate, and protease inhibitor mix (Life)) by 30 min shaking at 4°C, while the supernatant was ultracentrifuged (30 min, 55,000 rpm, 4°C). The supernatant from the ultracentrifugation was collected as DEA fraction, and the pH was adjusted by adding 10% 0.5 M Tris/HCl pH 6.8. The RIPA lysates in the Precellys tubes were centrifuged at 4,000 g (10 min at 4°C). The remaining pellet was homogenized in 60-70 µl of 70% formic acid (FA) (Merk) while the supernatant was combined with the pellet from the first ultracentrifugation after solubilizing with 50 µl of fresh RIPA buffer. The combined RIPA lysates were subjected to ultracentrifugation (60 min, 55,000 rpm, 4°C), and the supernatant was collected as RIPA fraction. The FA lysate was sonicated for 7 min in a bath-sonicator (Sonorex RK100H, Bandelin), centrifuged at 20,000 g (30 min at 4°C). The supernatant (20 µl) was neutralized with 380 µl 1 M Tris-HCl buffer at pH 9.5, and used as FA fraction. Total protein concentrations in DEA and RIPA fractions were determined using the BCA assay (Interchim) and adjusted by each buffer before further analysis. Concentrations of FA fractions were adjusted based on those of RIPA fractions.

### Western Blot

For SDS-PAGE analysis, proteins were mixed with 4x Laemmli Sample Buffer (Bio Rad) containing 2-mercaptoethanol and boiled for 5 min at 95°C. After separating the proteins on Tris-Tricine gels (10-20%, Thermo Fisher Scientific) with the XCell SureLock Mini-Cell system (Novex), proteins were transferred to nitrocellulose membranes (Amersham^{™} Protran^{®} Western blotting membranes, 0.2 µm, Merk) and boiled for 5 min in PBS. The membranes were then incubated with a blocking solution containing 0.2% I-Block (Thermo Fisher Scientific) and 0.1% Tween 20 (Sigma) in PBS for 1 h, followed by overnight incubation with primary antibodies in the blocking solution. The rat monoclonal 2D8 antibody against Aβ was described before. Other primary antibodies include anti-amyloid precursor protein [Y188] (abcam), anti-β-actin (Sigma), anti-APOE (mouse) (Thermo Fisher Scientific), anti-APOE (human) (Millipore), anti-Halo (Promega), anti-tau [Tau-5] (Thermo Fisher Scientific), anti-p-tau (Thermo Fisher Scientific), anti-ferritin heavy chain (abcam) antibodies. After removal of the antibody, membranes were washed three times in TBS-T (0.2% Tween 20, Sigma) buffer, subsequently incubated with secondary HRP antibodies (Thermo Fisher Scientific) for 1 h at room temperature, and washed three times again with TBS-T. To perform ECL detection, horseradish peroxidase substrate (ECL, GE Healthcare or ECL Plus, Thermo Scientific) was added to the membranes, and the resulting signals were captured on X-ray films (Super RX Medical X-Ray, Fujifilm). These films were then developed using an automated film developer (CAWOMAT 2000 IR, CAWO). For Native PAGE analysis, proteins were mixed with Novex^{™} Tris-Glycine Native Sample Buffer (2X) (Invitrogen) and loaded onto Tris-Glycine gels (4-12%, Thermo Fisher Scientific) with the XCell SureLock Mini-Cell system (Novex). Proteins were transferred to pre-activated PVDF membranes (Invitrolon^{™} PVDF / Filter Paper Sandwiches, 0.45 µm, Invitrogen) and boiled for 5 min in PBS before the incubation with the blocking solution. Antibodies used were listed in Key Resources Table.

### Isolation of sarkosyl-insoluble fractions and PBS extracted lysate

Sarkosyl-insoluble fractions from mouse brains were extracted as previously described with some modifications and used for Western Blot and stereotactic injection. Briefly, 20-40 mg of pulverized mouse cortices in Precellys tubes (Bertin Technologies) was homogenized in 10 vol (w/v) A68 extraction buffer (10 mM Tris-HCl, pH 7.5, 0.8 M NaCl, 10% sucrose and 1 mM EGTA) using a sample homogenizer (6,500 rpm. for 30 s at 4°C, Bertin Technologies). Homogenates were brought to 2% sarkosyl and incubated for 30 min at 37° C. Following a 10 min centrifugation at 10,000 g, the supernatants were spun at 113,000 g for 20 min at 25 °C. The pellets were washed with saline and ultracentrifuged as before. The resulting pellets were resuspended in 30 µl PBS for stereotaxic injection or in 30-50 µl 30 mM Tris-HCl (pH 7.4) for Western Blot. After 15 s sonication, the solutions were centrifuged at 1,000 g for 5 min at 4°C, and the supernatant was collected as sarkosyl-insoluble fractions. For stereotactic injections, Aβ in sarkosyl-insoluble fractions from 12-month-old *5xFAD* and 12-month-old *5xFAD ApoE* KO mice were quantified by Western Blot, and adjusted to the same concentration. Synthetic beta-amyloid peptide (1-42) (abcam) was used as the standards for the quantification. Preparation of PBS extracted lysate was conducted as previously described. Briefly, 30 mg of pulverized mouse cortex, derived from 12-month-old *5xFAD* mice, was homogenized in 10 vol (w/v) sterile PBS using a sample homogenizer (6,500 rpm. for 30 s at 4°C, Bertin Technologies) and sonicated for 15 s (30% amplitude, Digital Sonifier W-250D, Branson). The crude brain homogenate was centrifuged for 5 min (at 3,000 g, 4°C), and the supernatant was stored at -8o°C until use.

### APOE aggregate isolation from ApoE-Halo 5xFAD mouse and human brain

For mouse APOE aggregate isolation, the isolation of sarkosyl-insoluble fraction was conducted as described above. In order to increase the aggregate concentration, cortices from 2-4 *ApoE-Halo 5xFAD* mice (5-6-month-old) were pooled and homogenized in 5 vol (w/v) extraction buffer (10 mM Tris-HCl, pH 7.5, 0.8 M NaCl, 10% sucrose and 1 mM EGTA) using a sample homogenizer (6,500 rpm. for 30 s at 4 °C, Bertin Technologies). The pellets from ultracentrifuge after saline wash were resuspended in 200 µl 30 mM Tris-HCl (pH 7.4), sonicated by bath-sonicator (Sonorex RK100H, Bandelin) for 3 min, and centrifuged at 1,000 g for 5 min at 4 °C. Purification of APOE aggregates from the sarkosyl-insoluble fraction was conducted by immunoprecipitation using Halo-Trap Magnetic Agarose (Chromotek) according to manufacturer's instruction. After 2:3 dilution with a dilution buffer (10 mM Tris/Cl pH 7.5, 150 mM NaCl, 0.5 mM EDTA), sarkosyl-insoluble fractions were loaded onto the agarose beads and incubated overnight at 4 °C with continuous mixing. Beads elution was carried out by adding 200 mM glycine pH 2.5 to the beads and followed by the addition of 10% volume of neutralizing buffer (1M Tris, pH 10.4). The solution was then dialyzed against PBS by using Slide-A-Lyzer^{™} Dialysis Cassettes (Thermo Fisher Scientific, 10kDa). The dialyzed solution was collected, snap-frozen and stored at -8o°C as mouse APOE aggregates. For human APOE aggregate isolation, 200 mg of temporal cortex from Alzheimer's disease patient brain was homogenized in 20 vol (w/v) extraction buffer (10 mM Tris-HCl, pH 7.5, 0.8 M NaCl, 10% sucrose and 1 mM EGTA) using a sample homogenizer (6,500 rpm. for 30 s at 4 °C, Bertin Technologies). After addition of sarkosyl (2% as the final concentration), lysates were incubated for 24h at 37 °C with shaking. The lysates were then centrifuged at 10,000g for 10 min, and the supernatant was further ultracentrifuged 113,000 g for 20 min at 25 °C. After washing the pellet with saline, the pellet was solubilized in 30 mM Tris-HCl by pipetting and sonication for 3 min by bath-sonicator. The debris were removed by centrifuge at 1000 g for 5 min. The sarkosyl-insoluble fractions were incubated overnight with 1:25 volume of biotinylated APOE antibody (Novus Biologicals) at 4 °C with agitation at 400 rpm. Immunoprecipitation by streptavidin-conjugated magnetic beads, Dynabeads^{™} M-280 Streptavidin (Thermo Fisher Scientific), was performed according to the manufacturer's instructions. For Aβ immuno-depletion, the elute was mixed with 1:75 volume of biotinylated anti-β amyloid, 1-16 antibody (BioLegend) and incubated overnight at 4 °C with agitation at 400 rpm. After removal of antibody-bound Aβ by Dynabeads^{™} M-280 Streptavidin (Thermo Fisher Scientific), the flow-though was again added with 1:75 volume of biotinylated anti-β amyloid, 1-16 antibody, and incubated at 4 °C for 6h with agitation. The flow-through from the second immuno-depletion was then dialyzed overnight against PBS by using Slide-A-Lyzer^{™} Dialysis Cassettes (Thermo Fisher Scientific, 10kDa). The solution after dialysis was centrifuged at 1,000 g for 10 min at 4°C, and the supernatant was stored at -8o°C as human APOE aggregates. For both mouse and human APOE aggregates, the enrichment of APOE and absence of Aβ were analyzed by Western Blot while the aggregation status was confirmed by Native-PAGE and EM. Antibodies used were listed in Key Resources Table.

### Isolation of brain-derived soluble APOE-Halo from ApoE-Halo mouse

Purification of soluble APOE-Halo from *ApoE-Halo* mice was enabled through immunoprecipitation of Halo-tagged proteins using Halo-Trap Magnetic Agarose (Chromotek). Three 4-month-old *ApoE-Halo* mice were perfused with PBS, and the brainstems were transferred to Precellys tubes (Bertin Technologies). The brains were homogenized in 2 vol (w/v) RIPA buffer (20 mM Tris/HCl pH 7.5, 150 mM NaCl, 2.5 mM EDTA, 1% NP-40, 1% sodium deoxycholate, 2.5 mM sodium pyrophosphate, 1mM PMSF, and protease inhibitor mix (Life)) using a sample homogenizer (6,500 rpm. for 30 s at 4°C, Bertin Technologies). The lysates were then centrifuged at 17,000g for 20 min at 4°C, and the supernatant was diluted 2:3 with a dilution buffer (10 mM Tris/Cl pH 7.5, 150 mM NaCl, 0.5 mM EDTA) containing 1mM PMSF and protease inhibitor mix (Life). The diluted lysates were incubated overnight with the agarose beads at 4°C with continuous mixing. Beads elution was performed with 200 mM glycine pH 2.5, and the elute was dialyzed overnight against PBS by using Slide-A-Lyzer^{™} Dialysis Cassettes (Thermo Fisher Scientific, 10kDa). After centrifuge at 1,000g for 10 min (4°C), supernatant was collected as brain-derived soluble APOE-Halo and stored at -8o°C until use.

### Stereotactic injection of sarkosyl-insoluble fractions, PBS-extracted lysate, and APOE aggregates (mouse and human)

Animals were anesthetized with MMF (medetomidine 0.5 mg/kg, midazolam 5.0 mg/kg fentanyl 0.05 mg/kg). The mice were placed in a stereotactic frame and, after incision of the skin, small holes were drilled in the skull at the appropriate locations. The following coordinates were used to target prefrontal cortices unilaterally or bilaterally: AP + 2 mm, ML ± 0.55 mm, DV 1.8 mm (relative to Bregma). Each injection was performed with the speed of 150 nl/min, and the needle was left in situ for 5 mins before being slowly withdrawn. The injection volume per hemisphere was 1.5 µl for all the injection materials except TMR-Cholesterol (1.0 µl, 10 ng of TMR-Cholesterol in 10% DMSO in PBS). After surgery, the skin was closed with silk sutures, and the animal was put on a warm pad to prevent hypothermia.

### Immunohistochemistry and immunocytochemistry

Mice were anesthetized using ketamine/xylazine, perfused with PBS and the extracted tissue was postfixed with 4% paraformaldehyde (PFA) for 4-6 h, cryoprotected in 30% sucrose for 3 nights and subsequently embedded in Tissue-Tek OCT cryo embedding matrix followed by freezing the tissue on dry ice. Brain samples were cut in 16 µm sections (CryoStar NX70 HOMVP) and stored at -80°C. Immunohistological staining was done on sections that were adjusted to RT for 30 min and washed with PBS to remove the excess embedding matrix. For all immunofluorescent staining except Aβ labeling, sections were transferred in citrate buffer (0.01 M, pH 6.0) for antigen retrieval. Tissue permeabilization was performed by incubating sections with 0.5% Triton X100 for 10 min. For immunofluorescence detection, blocking was performed using a mixed blocking solution (2.5% bovine serum albumin, 2.5% fish gelatin, 2.5% fetal calf serum in PBS). For staining procedures requiring mouse antibodies, sections were additionally blocked with 1:100 mouse Fab fragment blocking (715-007-003, Jackson ImmunoResearch) in PBS for 1 h. Primary antibodies were anti-Aβ 1-11 (Biolegend), anti-Aβ 2D8, anti-Aβ 6E10 (Biolegend), anti-Aβ D3E10 (Cell signaling), anti-Aldh1l1 (Sigma), anti-BCAS1 (Novus Biologicals), anti-CD68 (Bio-Rad), anti-GFAP (Synaptic Systems), anti-IBA1 (Synaptic Systems), anti-NeuN (Millipore), anti-LAMP1 (Santa Cruz), anti-PDFGRa (R&D systems), anti-STAT1 (CST) diluted in antibody diluent (0.625% bovine serum albumin, 0.625% fish gelatin, 0.625% fetal calf serum in PBS) and incubated for 24-72 h followed by incubation with fluorophore-coupled secondary antibodies Alexa488 Donkey α-chicken (Jackson), Alexa488 Donkey α-guinea pig (Sigma), Alexa488 Donkey α-rat (Invitrogen), Alexa555 Donkey α-rat (Thermo), Alexa647 Goat α-mouse (Thermo), Alexa647 Donkey α-rabbit (Thermo), Alexa647 Donkey α-rat (Jackson), for 1 h at RT. APOE-Halo was detected by TMR Halo ligand (Promega), and ligand staining was implemented in the secondary antibody staining step. To visualize β-sheet structure, either MXO4, ThiazineRed, or Thioflavine S was used. For MXO4 (Tocris) staining, tissue was incubated with 4 µg/ml MXO4 in 50% ethanol for 30 min and washed for 5 min with 50% ethanol followed by three times wash with PBS. Thiazine Red (Sigma) was used in 1:1000 dilution in PBS for 15 min and washed three times with PBS. Thioflavine S (Sigma) was stained by using 1% Thioflavine S filtered solution and incubated for 8 min. Excess dye was removed by 2 x 3 min wash with 80% ethanol, 1×3 min wash with 95% ethanol, and 3 × 3 min wash with distilled water. After β-sheet structure staining, nuclear labeling was performed using either Hoechst (Thermo Fisher) or NucRed (Invitrogen). For human staining, blocking of endogenous peroxidase activity with 3% hydrogen peroxide was performed, which was followed by antigen retrieval with citrate buffer (0.01 M, pH 6.0) and blocking with above-mentioned mixed blocking solution. Incubation of three different primary antibodies and corresponding secondary antibodies was performed individually in different steps. Namely, incubation with first primary antibody anti-APOE 8D4 for 24 h was followed by HRP-coupled secondary antibody (Goat α-rabbit, Thermo) incubation for 1 h at RT and subsequent binding with Opal dye 570 (Perkin Elmer). Repetitive boiling step with citrate buffer as described was performed before incubating with the next primary antibody anti-Aβ1-11 (Biolegend) for 24 h, which was then followed by HRP-coupled secondary antibody (Thermo) incubation for 1 h at RT and subsequent binding with Opal dye 620 (Perkin Elmer). Subsequent to the last boiling step with citrate buffer as described, the third primary antibody incubation anti-IBA1 (Synaptic Systems) for 24 h was followed by HRP-coupled secondary antibody (Invitrogen) incubation for 1 h at RT and subsequent binding with Opal dye 520 (Perkin Elmer) Serial antibody staining was followed by MXO4 staining and nuclear labeling as described previously. For immunocytochemistry, cells were stained after 1 min permeabilization with 0.1% Triton and 1 h blocking with previously described blocking solution. Primary antibody incubation was performed overnight and secondary antibody incubation for 1 h at RT. After removal of secondary antibody, cells were subject to MXO4 and nuclear labeling as described earlier.

### Image acquisition and quantification

Optical sections were acquired with a confocal laser-scanning microscope (Zeiss LSM 900 AiryScan) using a LD C-Apochromat 40x/1.1 Water objective (Zoom factor 0.5 -1.3) or objective Plan-Apochromat 63x/1.4 Oil DIC M27 (Zoom factor 1.3-2.5). Z-step was kept between 0.8 to 1.5 µm for quantitative imaging and 0.22 µm for 3D reconstruction imaging. Images were acquired in bidirectional mode (Aryscan Mode SR:4.1 2D Auto) and LSM scan speed was kept between 5 to 7. For the 3D reconstructions, 3D images were first generated from the individual z-planes in the Surpass view option of Imaris x64 (version 9.2.0, Bitplane). The region of interest was then segmented and a "Surface" was created for each channel, using the Surface creator tool. For immunohistochemistry analysis two to three sections from each animal were analyzed using Image J software. For injection experiments, tile scan images at the injection site identified by IBA1 reactivity were analyzed by fixed threshold method and particle analyzer (Size 0.2 µm² -infinity, circularity 0-1). Quantification of MX04+ extracellular plaques and intracellular MX04+ aggregates were performed on single z-planes using cell counter plug in. Mean size of 25-30 microglial (IBA1+) intralysosomal (CD68+) MX04+ aggregates per mouse in *APP*/*PS1* and *APP*/*PS1 Trem2 KO* mice were quantified by fixed threshold method on consecutive single z-planes. MX04+ aggreges in cell culture experiments were quantified by fixed threshold method and particle analyzer (Size 0.01 µm² -infinity, circularity 0-1).

### Light sheet microscopy

After perfusion with 4% PFA, immersion fixed brain hemispheres were processed for whole mount tissue Congo red labelling and subsequent clearing following a modified iDISCO protocol. Hemispheres were dehydrated using subsequent steps in 50% methanol, 80% methanol, and twice 100% methanol, followed by a bleaching step using H₂O₂:DMSO:methanol in a 1:1:4 ratio. Lipids were removed with three consecutive 100% methanol steps and one 80% methanol step and rehydrated with 80% methanol, 50% methanol and 100% PBS and two consecutive steps of 0.2% Triton X-100. Hemispheres were permeabilized by incubating in 0.2% Triton with additional 20% DMSO and 0.3 M glycine, followed by twice 0.2% Tween20 plus 10 mg/ml heparin and 5mM sodium acide. Labelling with congo red takes place in 0.2% Triton with additional 20% DMSO and 0.3 M glycine for three days and washed 6 times with 0.2% Tween20 plus 10 mg/ml heparin and 5mM sodium acide, Afterwards brains were dehydrated with subsequent 20% , 40%, 60%, 100% methanol and delipidated with methanol:dichlormethane in a ratio of 1:2 followed by 100% dichlormethane. Before imaging the tissue is cleared using ethylcinnamate (Eci) and hemispheres were mounted onto the sample holder with the medial side down and placed into the imaging chamber filled with Eci to acquire sagittal imaging planes. Light sheet microscopy was performed using a LaVision Ultramicroscope II equipped with 2x objective, corrected dipping cap and zoom body. Images were acquired in the mosaic acquisition mode with the following settings: 5 µm light sheet thickness, 20% sheet width, 0.154 sheet numerical aperture, 4 µm z-step size, 1000 x 1600 px field of view, 4 × 4 tiling, dual light sheet illumination, and 100 ms camera exposure time. Red fluorescence was recorded with 561 nm laser excitation at 80% laser power and a 585/40 nm emission filter. Autofluorescence was recorded with 488 nm laser excitation at 50% and 525/20 nm emission filter. Images were imported into Vision4D v3.2 (Arivis) and stitched using the tile sorter setup. Partly, images were imported and stitched using Imaris Importer and Stitcher v9.1 (Bitplane). Rendered hemibrains were then processed and two main regions of interest were manually annotated based on the sagittal Allen mouse brain atlas, namely isocortex and hippocampus. All ROIs were first traced manually in 2D planes to automatically extrapolate the 3D ROIs. Next, we segmented amyloid plaques within the ROIs. Plaques were segmented using the blob finder algorithm in Vision4D with the following parameters: 20 µm object size, 10-15% probability threshold, and 0% split sensitivity. Once segmentation has been performed, a stringent noise removal was performed by deleting objects with voxel sizes < 10 from the object table. Additional noise due to blood vessels were removed manually.

### Preparation of monomeric/tetrameric recombinant APOE4

Isolation of monomeric / tetrameric APOE was performed as described. Briefly, N-terminally His-tagged recombinant APOE4, which was bacterially overexpressed and purified by Ni-IDA (> 90% purity and endotoxin < 0.1 EU/µg), was produced by BiologicsCorp. Lyophilized protein was reconstituted in a 4 M guanidine hydrochloride on ice and injected onto a Superdex^{™} 200 Increase 10/300GL column equilibrated in TBS (10 mM Tris, 150 mM NaCl, 0.25 mM EDTA, pH 7.4) for FPLC. The protein eluted as two peaks and proteins in each peak fraction were analyzed by Native-PAGE to confirm that the first and second peaks represented aggregated and monomeric / tetrameric APOE, respectively. Here, monomeric / tetrameric APOE was collected and snap-frozen in liquid nitrogen and stored at -80 °C until analysis. FPLC of monomeric / tetrameric APOE after a cycle of freeze / thaw showed no change in aggregation state when compared with the freshly prepared protein. Concentration of FPLC-purified monomeric / tetrameric APOE4 was determined by absorbance measurements at 280 nm using an extinction coefficient of 44,460 M⁻¹·cm⁻¹.

### Preparation of POPC/cholesterol-lipidated APOE

Lipidation of recombinant mouse APOE was performed as previously described. The mixture of Palmitoyl-2-oleoyl-sn-glycero-3-phosphocholine (POPC, Avanti Lipids) and unesterified cholesterol (Sigma) in a 90:5 molar ratio was comixed in chloroform and dried using a continuous flow of nitrogen gas in a glass vial. For the preparation of TMR-lipidated APOE-Halo, the same molar concentration of TopFluor^{®} TMR Cholesterol (Avanti) was used instead of untagged cholesterol. The resulting lipids were then suspended in sterile PBS to obtain a concentration of 5 mg lipids/ml PBS (5.11 mg lipids/ml PBS for TMR-lipidated APOE-Halo). The solution was vortexed thoroughly for 3 min and kept at RT for 30min. For TMR-lipidated APOE-Halo, liposome solutions were solubilized further by pipetting and 2-3 min sonication using bath-sonicator (Sonorex RK100H, Bandelin). Subsequently, sodium cholate (50 mg/ml, Sigma) was slowly titrated into the liposome solution (3 volumes of sodium cholate for 1 volume of lipids). After 5 min of gentle vortex, the solution was kept at RT for 30-60 min. Recombinant mouse APOE, human APOE3, human APOE4, or mouse APOE-Halo (BiologicsCorp) was then added to the liposome solution (APOE : POPC : cholesterol, molar ratio of 1 : 90 : 5) and mixed gently for 5 min by tapping. The solution was kept at room temperature for 1 h and dialyzed for 3 days against PBS at 4°C using Slide-A-Lyzer^{™} Dialysis Cassettes (Thermo Fisher Scientific, 10kDa). Lipidated APOE particles were analyzed by Native-PAGE, DLS, EM, and density gradient ultracentrifugation with Optiprep^{™} Density Gradient Medium (Sigma). APOE concentrations were determined by immunoblot, and samples were stored in -80°C until use.

### Analysis of intracellular APOE-Halo lipidation status by density ultracentrifugation

Primary microglia was treated with 0.2 µM POPC/Cholesterol-lipidated mouse APOE-Halo for 1h or 4h following overnight preincubation with serum-free medium. After washing the cells twice with PBS, cells were scraped and collected in 160 µl PBS without using detergent. Cells were then lysed through 3 cycles of freeze and thaw and centrifuged at 10.000g for 10 min at 4°C. The supernatant was collected as the cell extracts and subject to density ultracentrifugation by OptiPrep^{™} Density Gradient Medium (Sigma). In order to exclude the possibility of potential delipidation due to the lysis step, culture medium containing POPC/Cholesterol-lipidated mouse APOE-Halo was processed equally to cell extracts with the same cycles of freeze/thaw and the centrifuge. As preparation for density ultracentrifugation, cell extracts or medium were mixed with 60 % Optiprep in 2:3 ratio to reach 36% Optiprep concentration and transferred to an ultracentrifuge tube (Beckman Coulter) (Figure S7E). 24% and 0% Optiprep solutions diluted with 10mM HEPES (500 µl each) were layered on top, and ultracentrifugation was performed at 5,4000 rpm for 3 hours at 4°C. The POPC/cholesterol-lipidated APOE-Halo floated to the region between the 0% and 24% Optiprep. Practically, the top 500 µl was discarded as 0% Optiprep fraction, and the next 150 µl was collected as the floating fraction. After removing the residual 24% Optiprep solution (350 µl), the solution remaining in the bottom was collected as the non-floating fraction. Both floating and non-floating fractions were analyzed by SDS-PAGE.

### Real-time quaking-induced conversion (RT-OuIC) assay

Purified monomeric / tetrameric APOE4 was incubated with / without APOE aggregates from a human AD brain continuously for 12.5-18 days in the presence of 10 µM Thioflavin T (ThT). Monomeric / tetrameric APOE4 was prepared in two different conditions; 1) high concentration (500 µg/ml) and low volume (100 µl/sample), and 2) low concentration (220 µg/ml) and high volume (240 µl/sample). For comparison between unlipidated/lipidated APOE3 and APOE4, 14.2 µM (500 µg/ml) of purified monomeric/tetrameric APOE or the same molar concentration of POPC/cholesterol-lipidated APOE was incubated for 18-21 days with 10 µM ThT. All samples were added with 10% volume of 10 x PBS before the start of incubation. Black 96-well plates with a clear bottom (Corning) were sealed and incubated in FLUOstar Omega (BMG Labtech) plate reader at 37°C with intermittent double orbital shaking at 400 rpm for 1 min, followed by 1 min rest. ThT fluorescence was measured using 448 nm excitation and 482 nm emission filter, and the values from every 4 h were plotted.

### Circular dichroism (CD) spectroscopy

For chronological analysis of incubated APOE4, monomeric / tetrameric protein (0.15 mg/ml) was prepared in TBS and incubated at 37°C for various times. Circular Dichroism spectra were measured with a Chirascan V100 instrument (Applied Photophysics, UK). A cuvette with 0.2 cm path length was filled with 160 µl of the APOE4 solution. Measurements were performed at 25°C, 190 - 260 nm, with 4 repeats. Control spectra obtained with TBS buffer alone were subtracted from the experimental spectra. The measured values (mdeg) at different time points were plotted. For comparison between unlipidated/lipidated APOE3 and APOE4, 14.2 µM (500 µg/ml) of purified monomeric/tetrameric APOE or the same molar concentration of POPC/cholesterol-lipidated APOE was incubated for 3 weeks. The mean residue ellipticity ([Θ] in deg cm² dmol⁻¹) was calculated as previously described. Protein concentrations were measured by a NanoDrop spectrometer by measuring the absorbance at 280 nm using an extinction coefficient of 44,460 L mol⁻¹ cm⁻¹.

### Negative staining and transmission electron microscopy (TEM)

For transmission electron microscopic (TEM) observation, carbon coated copper grids (Science Services) were glow discharged for 30 sec in a Harrick plasma cleaner (PDC-32G-2) to facilitate adsorption. After fixing the grid by anti-capillary inverse tweezers (Dumont) a volume of 1.5 µl of the sample was deposited onto the grid for 2 min and blotted shortly using filter paper (Whatman). Negative staining was performed by addition of 1.5 µl 1% uranyl acetate in water for 30 s. After blotting, the grid was air dried for at least 30 min. Immunogold staining was performed on plasma-treated carbon coated nickel grids (Science Services). After adsorption of the APOE aggregates and washing, the samples were incubated in blocking buffer (5% acetylated BSA in distilled water). The primary antibodies (rabbit anti-Halo, 1:500, Promega, rabbit anti-APOE, 1:500, Thermo-Fisher) in blocking buffer were added for 90 min, washed three times in water and blotted. After secondary antibody (goat anti rabbit 10 nm gold, 1:20, Aurion) incubation for 90 min and three washing steps, the grid was contrasted in 1% uranyl acetate in water for 1 min the dark. The grids were blotted and air-dried for 30 min. Transmission Electron Microscopy micrographs were acquired on a JEM 1400plus (JEOL) equipped with a XF416 camera (TVIPS) and the EM-Menu software (TVIPS).

### Volume scanning electron microscopy

For correlative EM, mice were anesthetized using ketamine/xylazine and perfused with PBS, the extracted tissue was postfixed in the fixative (4% PFA, 2.5% glutaraldehyde in 0.1 M cacodylate buffer) for 24 h and processed by cutting 40 µm vibratome sections. Section were postfixed with 2.5% glutaraldehyde in 0.1M cacodylate buffer (Science Services) for EM analysis and subjected to a standard rOTO contrasting procedure. After postfixation in 1% osmium tetroxide (Electron Microscope Services), 1.5% potassium ferricyanide (Sigma-Aldrich) in 0.1M sodium cacodylate, staining was enhanced by reaction with 1% thiocarbohydrazide (Sigma-Aldrich) for 45 min at 40°C. The tissue was washed in water and incubated in 1% aqueous osmium tetroxide, washed and further contrasted by overnight incubation in 1% aqueous uranyl acetate at 4°C and 2 h at 50°C. Samples were dehydrated in an ascending ethanol series and infiltrated with LX112 (LADD). Tissue was serially sectioned at 100 nm thickness on to carbon nanotube (CNT) tape (Science Services) on an ATUMtome (Powertome, RMC) using a 35° ultra-diamond knife (Diatome). CNT tape stripes were assembled on to adhesive carbon tape (Science Services) attached to 4 inch silicon wafers (Siegert Wafer) and grounded by adhesive carbon tape strips (Science Services). EM micrographs were acquired on a Crossbeam Gemini 340 SEM (Zeiss) with a four-quadrant backscatter detector at 8 kV. Overview scans were taken at 200 nm lateral and higher resolution scans at 10 nm lateral resolution. Serial section data were aligned by a sequence of automatic and manual processing steps in Fiji TrakEM2 and relevant regions selected.

### Active immunization against fibrillary ApoE4

Immunization against ApoE4 aggregates was performed by subcutaneous (s.c.) immunization with 50 µg of aggregates ApoE4 in complete Freund's adjuvant (Mycobacterium tuberculosis at 3.75 mg/ml) and intraperitoneal injection twice with 500 ng of pertussis toxin (PTX). Complete Freund's adjuvant was prepared by adding M. tuberculosis to incomplete Freund's adjuvant consisting of paraffin oil and mannide mono-oleate as a surfactant (85%/15%). To obtain ApoE4 aggregate emulsion, equal volumes of ApoE4 aggregates in 1xPBS and CFA were mixed using a 3-way valve and syringes with Luer lock system. ApoE4 aggregate emulsion was prepared on the day of immunization. The emulsion was injected (s.c.) to both inguinal and axillary regions. PTX was injected directly after injection of ApoE4 aggregate emulsion and 2 days later.

### Statistics

Data are shown as mean ± SEM. No statistical methods were used to predetermine sample sizes, but the sample sizes are similar to those generally employed in the field. Each dot represents one animal. Normal distribution of the samples was tested using the Shapiro-Wilk test. For statistical analysis, paired or unpaired Student's t-test, or if appropriate the Mann-Whitney U-test, was used to compare two groups. Two-sided, one-way analysis of variance (ANOVA) followed by post hoc Tukey's test was used for multiple comparisons. Tests were chosen according to their distribution. In all tests, a P value < 0.05 was considered significant with *P < 0.05, **P < 0.01 and ***P < 0.001. Statistical analyses were done using GraphPad Prism (GraphPad Software, Inc.). Data acquisition and analysis were performed in a blinded manner. No animals were excluded from the analyses.

### Example 2

All methods mentioned in this example were carried out as described in Example 1.

### Generation and characterization of transgenic mice expressing the Halo-Tag protein domain fused to endogenous APOE

Visualization and purification of APOE has been restricted by relatively poor sensitivity and specificity of anti-APOE antibodies. To overcome this limitation, the present inventors have taken advantage of the Halo Tag self-labeling protein technology for detection and biochemical purification of endogenous protein. Transgenic knock-in mice expressing an APOE-HaloTag fused to the carboxyl terminus of full-length mouse APOE were generated by CRISPR/Cas9 mediated gene editing (APOE-Halo). Previous studies have demonstrated that the fusion of a C-terminal tag to APOE does not disrupt its biological functions, which is secretion, lipidation, and lipoprotein assembly.

Plaque pathology of Aβ can be determined by histologic labeling of fibrillar β-sheet deposits using Methoxy-X04 (MX04). The fluorescent HaloTag ligand was used to determine the cellular localization of APOE-Halo in *5xFAD* mice. The APOE-Halo ligand labeling was located to fibrillar β-sheet positive deposits (MX0₄⁺) in cortical tissue samples (Figure 1A).

### Example 3

All methods mentioned in this example were carried out as described in Example 1.

### APOE aggregation in 5xFAD and human AD autopsies

The HaloTag self-labeling protein technology enabled the determination of the APOE localization in Aβ amyloid plaques with high sensitivity and specificity. As expected, the majority of MX04⁺ plaques were positive for Aβ and APOE labelling (ApoE/Aβ intense plaques) at 3.5 months (82.1 ± 0.7 SEM %) and 6 months (68.6 ± 6.0 SEM %) in *5xFAD ApoE-Halo* mice (Figure 1B). With disease progression, an increase in fibrillary Aβ plaques was noted, which are positive for MX04 but with low labelling intensity for APOE (Aβ intense plaques) (increasing from 4.1 ± 0.4 SEM % at 3.5 month to 19.7 ± 3.4 SEM % at 6 months). Surprisingly, an additional type of MX04⁺ deposits was detected. These were fibrillar in appearance, but without detectable Aβ as determined with various antibodies. Instead, they were strongly positive for APOE-Halo ligand (APOE intense plaques). Quantification revealed that these MX04⁺ structures occurred already early in the disease process and remained relatively stable over time with a proportion of 14 ± 1.0 SEM % at 3 months of age and 11 ± 2.2 SEM % at 6 months (Figure 1B, 1C, and 1D). To rule out unspecific MX04 labeling, two additional β-sheet-binding dyes were used, Thiazine red and Thioflavin, which confirmed the presence of APOE intense β-sheet-positive structures.

Intracellular MX04⁺ aggregates within microglia were not specific to the *5xFAD* AD model, as they were also detected in human autopsy samples (Figure 2).

Due to the presence of dense APOE-rich fibrillar β-sheet positive deposits with different levels of Aβ immunostaining the relationship between APOE and Aβ aggregates in *vivo* was determined. APOE and Aβ may co-aggregate into one unified structure or remain separated as two distinct aggregates. The present inventors developed a specific biochemical purification method based on the sarkosyl extraction method optimized for Aβ fibril isolation (Figure 3A). The detergent-insolubility of protein aggregates was taken advantage of, and 2% sarkosyl-insoluble *5xFAD ApoE-Halo* brain lysate fractions were prepared. As shown by Western blotting both APOE and Aβ were recovered from the sarkosyl-insoluble fraction. Furthermore, it was found that protein extraction with higher sarkosyl concentration enriches APOE and Aβ content suggesting the high insolubility of plaque-associated APOE (Figure 3D). Next, the Halo-Trap Magnetic Agarose immune isolation system was applied, to purify APOE aggregates. Strikingly, APOE-Halo but not Aβ was detected in the immunopurified sarkosyl-insoluble faction, suggesting that APOE does not co-deposit with Aβ into conjoined insoluble protein aggregates (Figure 3B, 3E). Native-PAGE was used and it was found that purified APOE-Halo aggregates from sarkosyl-insoluble fractions consisted as multimers after isolation, which was in contrast to the heterogenous soluble APOE-Halo species purified from *ApoE-Halo* mice (Figure 3F). EM revealed wavy snake-like fibrillar morphology, consistent with previous reports from recombinant APOE that were positive for ApoE immuno-gold labelling (Figure 3A, 3C). In addition, isolated APOE-Halo aggregates showed MXO4 positivity after addition to microglial cell culture (Figure 3I, Figure 1B). To determine whether APOE aggregates can also be purified from human AD brain autopsy samples, a sarkosyl extraction and immunopurification protocol from AD cortex was established using 2% sarkosyl and antibodies against human APOE and Aβ (Figure 3A). Immunopurification of sarkosyl-insoluble APOE was followed by immunodepletion using Aβ antibodies allowing isolation of APOE aggregates with higher purity (Figure 3B, 3G, 3H). APOE but only minimal amounts of Aβ were detected, and negative staining revealed wavy snake-like fibrillar morphology of the isolated aggregates, as observed in mice that were positive for APOE immune-gold labelling (Figure 3A, 3C).

### Example 4

All methods mentioned in this example were carried out as described in Example 1.

### APOE aggregates act as a co-factor in β-amyloid pathology initiation

To determine whether the purified APOE aggregates can facilitate Aβ plaque formation experiments were conducted seeding, which is an established tool to identify co-factors in plaque generation. The present inventors made modifications to the previously reported protocols by conducting injections into the prefrontal cortex of *5xFAD* mice, wherein the seeding material was applied to one hemisphere and PBS to the other hemisphere, allowing to obtain quantitative comparisons with a relatively low number of animals. The injections were administered at the onset of amyloid plaque formation (~8 weeks), and analysis was limited to 2 months post-injection to prevent potential cross seeding into the opposite hemisphere. First, the experimental protocol using *5xFAD* brain homogenates (PBS-extracted lysates) was established, which has previously been shown to trigger Aβ plaque formation, and was compared with the injection of sarkosyl-insoluble *5xFAD* brain fractions. Both fractions led to enhanced seeding as determined by the ~2-fold increase in MXO4⁺ structures compared to the PBS injected hemisphere (Figure 4A). Next the seeding activity of immunopurified APOE aggregates purified from the sarkosyl-insoluble *5xFAD ApoE-Halo* brain fractions was determined. First, immunopurified APOE aggregates were injected into a wild type mouse brain that led to the detection of few tiny MXO4⁺ fibrils at the injection site that were negative for Aβ (data not shown), providing evidence for the strong enrichment of APOE in the aggregates (see also Figure 3B, 3I). Analysis of *5xFAD* mice injected with immunopurified APOE aggregates unveiled a significant elevation in number of MX04⁺ structures (Figure 4B). MXO4⁺ signals colocalized with Aβ with compacted morphology indicating plaque generation (Figure 4B).

To gather additional evidence regarding the role of APOE aggregates as a co-factor in Aβ amyloid plaque formation, injection experiments were performed using sarkosyl-insoluble material derived from *5xFAD* and *5xFAD ApoE KO* mice. Because Aβ plaque formation is reduced and occurs later in the absence of APOE, 12-month-old *5xFAD ApoE KO* mice were used for the sarkosyl extractions and, in addition, the concentration of the extracts was adjusted to ensure that Aβ concentrations were comparable (Figure 4C). Injections using sarkosyl-insoluble material isolated from *5xFAD* resulted in substantial MXO4⁺ fibrillary aggregate formation (~2.8 ± 0.5 SEM fold to control). Strikingly, when injections were performed with sarkosyl-insoluble material isolated from *5xFAD ApoE KO* mice, no evidence of plaque seeding in *5xFAD* mice was found (Figure 4C). Together, these experiments provide evidence for sarkosyl-insoluble APOE as a co-factor in β-amyloid plaque generation.

### Example 5

All methods mentioned in this example were carried out as described in Example 1.

### Human APOE aggregates act as a co-factor in β-amyloid plaque formation

To investigate the relevance of the finding for human AD, a comparison of APOE3 and APOE4 was carried out. First, humanized APOE3 and APOE4 knock-in mice (*APOE3 KI,* APOE*4 KI*) were employed, which were crossbred with *5xFAD* mice. MX04⁺ amyloid plaque numbers were compared in *5xFAD, 5xFAD* APOE3 *KI, 5xFAD* APOE4 *KI* and *5xFAD ApoE KO.* Higher MX04⁺ plaque loads were detected in *5xFAD APOE4 KI* as compared to *5xFAD APOE3 KI* and *5xFAD* mice (Figure 5A, 5B). Next, it was investigated whether APOE aggregates isolated from brain autopsy from AD patients either homozygous for APOE3 or APOE4 (obtained from Aβ immunodepleted sarkosyl-insoluble human AD autopsy samples, see Figure 3H) were able to act as a co-factor in β-amyloid plaque formation. Immunopurified human APOE3 and APOE4 aggregates were injected into *5xFAD* mice, and it was found that both were able to enhance MX04⁺ plaque formation as compared to control injections (Figure 5D). Subsequently, the experiment were carried out by injecting purified human AD derived APOE aggregates from the same patient into either *5xFAD* APOE3 *KI* or *5xFAD* APOE4 *KI* mice. It was found that APOE aggregate injections increased amyloid plaque numbers compared to control injections in both mouse lines, with higher MXO4⁺ plaque counts observed in *5xFAD* APOE4 *KI* mice (Figure 5C). Together, these experiments show that both APOE3 and APOE4 can act as a co-factor in β-amyloid plaque formation. To determine potential differences between the two APOE isoforms, it was investigated whether there are differences in their ability to aggregate *in vitro* and to function as seeds in *vivo.* Monomeric / tetrameric species of recombinant human APOE3 and APOE4 were purified via FPLC and conformational changes were analyzed over the incubation at 37°C for up to 4 weeks (Figure 6A, 6B, 6C). FPLC analysis detected the emergence of APOE4 in the higher molecular weight fractions indicating aggregate formation. To analyze how APOE4 changes its conformation, real-time quaking-induced conversion (RT-QuIC) was performed. Analysis revealed increasing Thioflavin T fluorescence intensity of APOE4, which reached a plateau after 2 weeks (Figure 6D). In addition, abundance of α-helical structures and θ222/θ208 ratio were examined by circular dichroism (CD) spectroscopy, which showed markedly reduced α-helicity and loss of paired-coil structures post-incubation (Figure 6E). Finally, ultrastructural analysis of APOE4 aggregates after 2-week incubation showed clusters of wavy fibrillar structures resembling in *vivo* isolated APOE4 aggregates (Figure 6F, 3A). Surprsingly, when APOE3 was analyzed similarly, no altered θ222/θ208 ratio, no increase in Thioflavin T fluorescence intensity or drastic change in molecular weight determined by FLPC after a 3-week incubation was observed (Figure 6G, 6H). In addition, when the assay was performed with APOE3 and APOE4 prepared as lipidated particles, no aggregation was detected indicating that lipidation hampers aggregation (Figure 6I, 6J). To determine, whether such in vitro-generated recombinant APOE4 aggregates can serve as a co-factor in amyloid plaque formation in *vivo,* recombinant APOE4 aggregates were injected into *5xFAD* mice and the MXO4⁺ plaque number was quantified (Figure 6K right panel, 6L). A significant increase of MXO4⁺ amyloid plaques was detected. Injection of recombinant APOE3, which was prepared similarly to *in vitro* generated recombinant APOE4 aggregates via a 3-week preincubation, did not result in an increase in the number of MXO4⁺ amyloid plaques in the seeding experiment (Figure 6K left panel). Together, these findings show that *in vitro-*derived human APOE4 aggregates can act as a co-factor in β-amyloid plaque formation similarly to in vivo-purified aggregates.

Moreover, the RT-QuIC assay with monomeric Aβ was performed, which resulted in rapid increase in Thioflavin T fluorescence intensity as expected. Co-incubating Aβ with 3-week preincubated recombinant human APOE (pre-aggregated APOE) significantly accelerated Aβ aggregation, indicated by a rapid increase in Thioflavin T fluorescence intensity and reduced lag time. APOE4 had a stronger effect on enhancing Aβ aggregation kinetics compared to APOE3 (Figure 6M).

Together, these findings show that in vitro-derived APOE aggregates can act as a co-factor in β-amyloid plaque formation similarly to in vivo-purified aggregates.

### Example 6

All methods mentioned in this example were carried out as described in Example 1.

### Microglia specific cholesterol synthesis ablation enhances aggregate formation

Under physiological conditions, APOE is associated with lipids. Thus, it was investigated whether lipidated APOE particles could form MXO4⁺ aggregate formation in microglia. In order to evaluate this, a comparison between lipid-free recombinant APOE and APOE associated with lipids (specifically phosphatidylcholine and cholesterol) was conducted along with Aβ-42, to generate aggregates in the cell culture aggregation assay. Surprisingly, APOE lipidation enhanced MXO4⁺ aggregate formation (Figure 7A). In addition, experiments performed in BV2 cells and primary microglia, showed that lipidated human APOE4 was more potent in inducing MXO4⁺ aggregates compared to lipidated human APOE3 (Figure 7A). Moreover, when uptake experiments using unlipidated and lipidated APOE together with Aβ-42 were performed, increased uptake of APOE was found when lipidated APOE was applied (Figure 7B). Thus, a possible reason for the facilitated aggregation due to APOE lipidation could be enhanced uptake. However, this may be followed by subsequent delipidation upon entering the endo-lysosomal compartment, thereby creating lipid-depleted, aggregation-prone species. To test this possibility, APOE-Halo lipoprotein particles were generated, together with a fluorescent cholesterol derivate (TMR-cholesterol). Using native gels, it was possible to demonstrate the stable incorporation of TMR-cholesterol into lipoprotein particles (Figure 7C). Next, uptake assays in primary cultures of microglia were performed, and demonstrating transfer of TMR-cholesterol to PLIN2⁺ lipid droplets already after 15min (Figure 7D), suggesting that TMR-cholesterol rapidly dissociated from APOE-Halo containing particle. Subsequently, a biochemical approach involving density gradient centrifugation was used to separate lipidated from unlipidated APOE-Halo after treating primary microglia with APOE-Halo lipoprotein particles. In the cell culture medium, APOE-Halo was detected within the lipid-rich fraction, while the majority of APOE-Halo was recovered from the lipid-depleted fraction in cell extracts. These data together indicate that APOE is taken up as a lipoprotein particle into cells, undergoes rapid delipidation, and persists in an unlipidated state within the cell for an extended period, creating a potential timeframe for aggregation.

In cell culture experiments in which the treatment of unlipidated with lipidated APOE together with Aβ-42 was compared, decreased expression of transcripts involved in cholesterol synthesis (*Hmgcr*, *Fdfti*, *Cyp51*, *Dhcr24*) was detected in cells treated with lipidated APOE (Figure 7F), indicating a feedback inhibition of the cholesterol synthesis pathway.

Given the experiments indicating a potential link between cholesterol metabolism, APOE uptake and aggregate formation in microglia, the next aim was to explore the possibility of leveraging this relationship for in *vivo* experiments. It was hypothesized that by reducing the synthesis of cellular cholesterol in microglia, there should be a subsequent increase in the expression of lipoprotein receptors, which, in turn, would augment the uptake of APOE and drive aggregate formation in microglia. Because suppression of sterol synthesis represent an established concept for promoting lipoprotein uptake, a strategy for blocking de-novo cholesterol synthesis in microglia was devised. The experiments were started in cell culture, and CRIPRS/Cas9 was used to generate BV2 cells that lack squalene synthase (*Fdft1*), an essential enzyme of the cholesterol biosynthesis pathway (Figure 7G). Ablation of *Fdft1* in BV2 cells was confirmed by real-time PCR expression analysis (*Fdft1* 0.01 ± 0.01 SEM fold to control). As predicted, *Fdtf1*-deficient cells cultured under low lipid conditions showed an upregulation of mRNA expression of several receptors related to APOE lipoprotein uptake (*Ldlr*, *Scarb1*, *CD36*), cholesterol usage (*Nceh1*) and reduced mRNA expression related to lipid efflux (*Abca1*, *Abcg1*)*.* Next, *Fdtf1*-deficient BV2 were used in comparison to control cells to determine whether APOE and Aβ mediated MX04⁺ aggregate formation is enhanced. Indeed, treatment with lipidated APOE and Aβ-42 was associated with a marked increase in MX04⁺ aggregate formation in *Fdtf1*-deficient BV2 as compared to control cells (5.6 ± 0.3 SEM fold) (Figure 7H).

The finding that inhibition of microglial cholesterol synthesis enhances MX04⁺ aggregation formation *in vitro,* resulted in an experimental strategy for cell specific genetic experiments in *vivo.* To this end *5xFAD* mice lacking a functional sterol synthesis pathway in microglia (*FDFT1 KO 5xFAD*; *CX3CR1CreERT2 Fdtf1*^{*flox*/}*^{flox} 5xFAD*) were generated, which were compared to *5xFAD* control mice (*CX3CR1Cre 5xFAD*; *CX3CR1CreERT2 Fdtf1*^{*wt*/}*^{wt} 5xFAD*)*.* 3D light sheet microscopy revealed a significantly augmented plaque load in *FDFT1 KO 5xFAD* mice at 3.5 months of age (Cortex: 6.9 ± 0.22 SEM fold to ctrl; Hippocampus: 1.6 ± 0.06 SEM fold to ctrl) (Figure 7I). In addition, enhanced numbers of microglia with MX04⁺ fibrillary morphology were detected within the lysosomal compartment (1.6 ± 0.02 SEM fold to control) following cholesterol synthesis ablation (Figure 7I), providing evidence that cholesterol-deprived microglia promote Aβ amyloidosis.

### Example 7

All methods mentioned in this example were carried out as described in Example 1.

### Microglial β-amyloid seeding requires APOE

To gain insights on the cellular and molecular factors that direct APOE to promote Aβ plaque formation, it was started by determining the localization of APOE-Halo⁺MX04⁺ aggregates in *5XFAD ApoE-Halo* mice using confocal Airyscan detector for improved resolution and signal-to-noise ratio. Surprisingly, by applying 3D rendering, marked labelling of APOE-Halo ligand and MXO4 within IBA1⁺ cells was noted (Figure 8B). Quantification revealed that a relatively large fraction of IBA1⁺ cells in the cortex were positive for both APOE-Halo and MX04 (84 ± 1.5 SEM %) (Figure 8B). APOE-Halo⁺MX04⁺ structures inside of IBA1+ cells were often associated with CD68, a marker for endosomal/lysosomal compartment of activated microglia (Figure 8B). To validate the high proportion of MX04⁺ structures in microglia, *5xFAD* mice independent of the APOE-Halo tag were analyzed, and comparable proportions of MX0₄⁺IBA₁⁺ cells were found in these mice (Figure 8C). Intracellular MX04⁺ aggregates within microglia were not specific to the *5xFAD* AD model, as these were detected in two additional mouse models of AD (*APP*/*PS1* and *NL-G-F* mice) (Figure 8E), as well as in human autopsy samples (Figure 8A). By volume scanning EM, lysosomal aggregates in fibrillary morphology within microglial cells were detected, particularly in the periplaque area of *5xFAD ApoE-Halo* mice (Figure 8D).

Next, the location where MX04⁺APOE⁺ aggregates originate from was investigated. One possibility is that they are first formed outside of microglia and subsequently internalized for degradation. Alternatively, MX04⁺ aggregates may develop inside microglia and then become released. To explore these possibilities, microglia were pharmacologically depleted using Plexxicon (PLX5622). The underlying rationale was that, even if both degradation and aggregation may occur simultaneously, the experiments should indicate which of these processes prevails. First, *5xFAD* brain lysates were injected into 8-week-old *5xFAD* mice that had been treated with PLX5622 or normal control chow, and the mice were analyzed 1-month post-injection to capture the MX04⁺ aggregate initiation phase. MX04⁺ aggregate formation was remarkably suppressed in PLX5622-treated mice as compared to the control-treated *5xFAD* mice (3.8 ± 1.1 SEM fold to control) (Figure 8F), providing evidence that MX04⁺ aggregate formation requires microglia. To elucidate the role of APOE in microglia-dependent MX04⁺ aggregate formation, immunopurified soluble APOE-Halo were injected into mice that had been treated with PLX5622 or normal control chow. The injections were administered to 8-week-old *5xFAD ApoE KO* mice, as these mice lack at this age almost entirely MX04⁺ aggregates (Figure 8G). The findings revealed that the injection of purified APOE-Halo led to the formation of MX04⁺ aggregates in control chow-treated *5xFAD ApoE KO* mice. This was in contrast to PLX5622-treated *5xFAD ApoE KO* mice, in which almost no detectable MX04⁺ aggregates were found (Figure 8G), providing evidence for the involvement of both APOE and microglia in MX04⁺ aggregate formation.

### Example 8

All methods mentioned in this example were carried out as described in Example 1.

### Lysosomal dysfunction and pro-inflammatory activation promote APOE-induced aggregate formation in microglia

It was explored whether APOE-dependent MX04⁺ aggregate formation can be reconstituted in cell culture. The microglial BV2 cell line was treated with recombinant APOE and synthetic fluorophore coupled Aβ-42, and aggregate formation was visualized using MX04. Immunocytochemistry revealed a concentration-dependent formation of MX04⁺ aggregates, which were positive for Aβ and APOE, 3 days after incubation (Figure 9A). Using the same treatment, MX04⁺ aggregate formation was also observed in primary microglia and human iPSC-derived microglia (Figure 9B). To determine the pathogenic relevance of MX04⁺ aggregates formed in cell culture, cell culture lysates were used for seeding experiments in *vivo.* Sarkosyl-insoluble APOE aggregates immunopurified from *5xFAD* mice and human AD brain samples, enhanced seeding of APOE and Aβ-42 dependent intracellular MX04⁺ aggregate formation in cell culture (Figure 9C).

As MX04⁺ aggregates were found within LAMP1⁺ endo-lysosomal organelles in *vivo* (Figure 8A-E), it was investigated how lysosomal function may modulate the aggregation process. Protein aggregation and degeneration may occur concomitantly within microglial lysosomes, and it was therefore hypothesized that interfering with lysosomal degradation may enhance aggregation. Indeed, when BV2 cells, primary microglia and human iPSC-derived microglia were treated with compounds that interfere with lysosomal degradative function (Lys05/chloroquine, bafilomycin or leupeptin), increased MX04⁺ aggregate formation was detected (Figure 9D, 9E). Next, the role of Triggering Receptor Expressed on Myeloid Cells 2 (TREM2) in MX04⁺ aggregate formation was explored, which initiates the disease-associated microglia (DAM) response program, associated with enhanced expression of genes with lysosomal degradative function. CRISPR-Cas9 was employed to generate TREM2-deficient BV2 cells, co-treatment experiments were conducted with APOE and Aβ-42, and an elevation in MX04⁺ aggregate formation in TREM2-deficient BV2 cells compared to control cells was observed (Figure 9D). To assess the in *vivo* relevance of these findings, MX04⁺ aggregates co-localizing with CD68 in IBA1⁺ cells in *APP*/*PS1 Trem2 KO* were quantified. Similarly to the results in cell culture, an increase in the area and number of MX04⁺ aggregates associated with CD68⁺ immunostaining in *APP*/*PS1 Trem2 KO* was observed compared to *APP*/*PS1* (Figure 9F)

Activated interferon-responsive microglia have been identified in models of AD, which coexists with DAM. Microglia in *5xFAD* mice were analyzed for expression of Signal transducer and activator of transcription 1 (STAT1 reactivity), a marker for interferon signal transduction activation. Co-labeling revealed that the majority of IBA1⁺ cells were positive for both STAT1 and MX04 in the cortex of *5xFAD* mice, irrespective of the disease stage (3 months: 82 ± 2.5 SEM %; 9 months: 77 ± 4.1 SEM %) (Figure 10A). In addition, STAT1⁺IBA1⁺ cells in direct contact to MX04⁺ plaques and in the periplaque area were detected. Next, it was explored whether JAK/STAT1 inhibition could modulate the generation of β-sheet-positive aggregates using the brain-permeable FDA-approved JAK/STAT1 inhibitor, baricitinib. 6-week-old *5xFAD* mice were treated with baricitinib (10mg/kg bodyweight) for 12 weeks, and plaque pathology was analyzed by 3D light-sheet microscopy using Congo red staining at 4.5 month of age. Remarkably, a significant reduction (45.8 ± 6.1 SEM %) of Congo red+ aggregates was observed as compared to untreated controls (Figure 10B). Moreover, a reduction in the number of STAT1⁺IBA1⁺ cells, and more importantly in the number of IBA1⁺ cells with MX04⁺ inclusions in *5xFAD* mice treated with baricitinib was found (Figure 10B). To further corroborate these findings, *5xFAD* mice lacking STAT1 (*5xFAD Stat1 KO*) were generated, and 3D light-sheet microscopy was performed using Congo red staining to determine the amyloid plaque load in the brain. A reduction of Congo red+ amyloid plaque number in 3.5-month-old *5xFAD Stat1 KO* compared to *5xFAD* mice was detected, validating the effects seen with baricitinib treatment (Figure 10C). Next, JAK/STAT1 signaling in cell culture was explored and it was found that the formation of MX04⁺ aggregates by APOE and Aβ-42 co-treatment was associated with STAT1 activation, visualized by its translocation into the nucleus (Figure 10D). Notably, baricitinib (1µM) potently reduced APOE and Aβ-42 mediated MX04⁺ aggregate formation in BV2 and primary microglia (Figure 10E). In addition, enhance MX04⁺ aggregate formation was enhanced by stimulating interferon signaling using interferon-γ (IFN-γ; 1ng/µl) (Figure 10D), which was reduced to control levels when cells were co-treated with baricitinib (Figure 10E).

### Example 9

All methods mentioned in this example were carried out as described in Example 1.

### Active immunization against fibrillary ApoE4 diminishes MX04+ plaques and reduces insoluble ApoE and Aβ

To explore the effect of an active immunization against fibrillary APOE4 (fAPOE4), *5xFAD* × APOE4KI mice at the age of 12 weeks were injected with 50µg of fApoE4 and the consequences 8 weeks post immunization were analyzed. Serum APOE levels of fAPOE4 immunized mice were unaffected compared to control mice but mouse IgG levels were increased in fAPOE immunized mice (Figure 11A). To determine the impact on plaque levels, brain tissue was fractionated in DEA, RIPA, and FA fractions to analyze Aβ and APOE levels in soluble and insoluble fractions. In immunized mice Aβ was reduced in the soluble DEA fraction as well as the FA fraction, whereas APOE is unaffected in the DEA fraction but reduced in the FA fraction. In addition, APP C-terminal fragments were attenuated in the RIPA fraction after immunization (Figure 11B). MX04+ plaques were analyzed to visualize the effect of the active immunization on the plaque load in different brain regions. MX04+ plaques were diminished in 5xFAD × APOE4KI mice 8 weeks after fAPOE immunization (Figure 11C).

As demonstrated by the results of the above Examples, the present inventors identified fibrillary aggregates of APOE, positive for β-sheets dyes, in mice with Aβ amyloidosis and in human AD brain autopsy samples. The present inventors were able to purify APOE aggregates, depleted of Aβ, from mice and human AD and could surprisingly show that they can serve as seeds for Aβ amyloidosis in mice. In addition, the results provide evidence that the aggregation process can occur within the endo-lysosomal system of microglia, and is modulated by immune and lipid metabolism.

The results surprisingly but convincingly show that immunization with recombinant fibrillary ApoE can efficiently reduce the load of soluble and insoluble Aβ. Also, insoluble forms of ApoE are reduced upon immunization with recombinant fibrillary ApoE. Moreover, immunization with fibrillary ApoE efficiently reduces fibrillary β-sheet deposits (MX04⁺ plaques) in the cortex and hippocampus.

Overall, the Examples of the present invention highlight the importance of fibrillary ApoE for the plaque formation and thus for neurodegenerative diseases, such as the Alzheimer's disease, and marks it as a novel therapeutic target for the treatment and/or prevention of neurodegenerative diseases. Thus, the immunization with fibrillary ApoE or the treatment with antigen-binding peptides specifically binding to fibrillary ApoE are important means for the treatment and/or prevention of neurodegenerative diseases, such as Alzheimer's disease.

## Claims

1. A fibrillary Apolipoprotein E (ApoE) for use in a method of treatment and/or prevention of a neurodegenerative disease in a patient in need thereof;
wherein, preferably, the fibrillary ApoE is a fibrillary recombinant ApoE, more preferably, a fibrillary recombinant human ApoE, most preferably a fibrillary recombinant human ApoE4.

2. The fibrillary ApoE for use according to claim 1, wherein the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. 1;
wherein, optionally, the ApoE is a fusion protein comprising one or more additional moieties or domains selected from a group comprising: affinity tags, linkers, and spacers, preferably, affinity tags and linkers, even more preferably affinity tags.

3. The fibrillary ApoE for use according to claim 1 or 2, wherein the ApoE is a fusion protein comprising an affinity tag selected from a group comprising a polyhistidine-tag, such as His6-tag, Halo-tag, Flag-tag, 3× Flag-tag, HA-tag, 3× HA-tag, c-Myc-tag, V5-tag, Strep-tag II (8 amino acids, WSHPQFEK-tag; SEQ ID NO:35), polyarginine tag (9 amino acids, RRRRRRRRR; SEQ ID NO:36), streptavidin binding peptide (SBP; 9 amino acids, AWRHPQFGG; SEQ ID NO:37), cellulose-binding tag (36 amino acids; QQTVWGQCGG QGWSGPTSCV AGSACSTLNP YYAQCI; SEQ ID NO:38), spot tag (12 amino acids, PDRVRAVSHW SS; SEQ ID NO:39), C-tag (4 amino acids, EPEA; SEQ ID NO:40), ALFA-tag (13 amino acids, SRLEEELRRR LTE; SEQ ID NO:41), Avi-tag (15 amino acids, GLNDIFEAQK IEWHE; SEQ ID NO:42), chitin binding protein (CBP), maltose binding protein (MBP), and gluthathione-S-transferase (GST); preferably a polyhistidine-tag, such as His6-tag, Halo-tag, Flag-tag, 3× Flag-tag, HA-tag, 3× HA-tag, c-Myc-tag, and V5-tag, even more preferably a polyhistidine-tag, such as His6-tag, and a Halo-tag; most preferably His6-tag;
wherein the affinity tag is fused to the N-terminus or the C-terminus of the ApoE sequence either directly or via a cleavable or non-cleavable linker, preferably the N-terminus.

4. The fibrillary ApoE for use according to any of the foregoing claims, wherein the ApoE is a fusion protein comprising an affinity tag, wherein the affinity tag comprises or consists of an amino acid sequence which is at least 90% identical, preferably at least 95% identical, more preferably at least 98% identical, even more preferably at least 99% identical, optionally identical, to an amino acid sequence selected from a group comprising SEQ ID No. 16, SEQ ID No. 17, SEQ ID No. 18, SEQ ID No. 19, SEQ ID No. 20, SEQ ID No. 21, SEQ ID No. 22, SEQ ID No. 23, and SEQ ID No. 24, preferably, SEQ ID No. 16 and SEQ ID No. 17, most preferably SEQ ID No. 16;
wherein, optionally, if the ApoE comprises a linker, the linker is either an amino acid-based linker, wherein the amino acid-based linker comprises or consists of an amino acid sequence which is at least 90% identical, preferably at least 95% identical, more preferably at least 98% identical, even more preferably at least 99% identical, optionally identical, to an amino acid sequence selected from a group comprising SEQ ID Nos. 25 to 34, or wherein the linker is a chemical linker, wherein the chemical linker is selected from a group comprising ValCitPABC linker, GGFG tetrapeptidyl-aminomethoxy linker, Aminoethyl-SS-ethylalcohol linker, Azido-Phenyl-Amido-S-S-Sulfo-NHS linker, Azidoethyl-SS-propionic NHS ester linker, Biotin-PEG4-S-S-acid linker, and Trifluoroacetamidoethyl-SS-propionic NHS ester linker.

5. The fibrillary ApoE for use according to any of the foregoing claims,
either wherein the ApoE is a fusion protein comprising a polyhistidine-tag, preferably His6-Tag, wherein the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. 2 or SEQ ID No. 3;
or
wherein the ApoE is a fusion protein comprising a Halo-tag, wherein the ApoE comprises or consists of an amino acid sequence which is at least 90% identical, preferably at least 95% identical, more preferably at least 98% identical, even more preferably at least 99% identical, optionally identical, to an amino acid sequence of SEQ ID No. SEQ ID No. 4 or SEQ ID No. 5.

6. The fibrillary ApoE for use according to any of the foregoing claims, wherein the fibrillary ApoE is produced in a method comprising the steps of:
i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence of SEQ ID No. 1, SEQ ID No. 2, SEQ ID No. 3, SEQ ID No. 4, and SEQ ID No. 5;
ii) cultivation of the suitable host cell under conditions that favor the production of the ApoE for an appropriate time period, wherein, optionally, the ApoE is secreted into the extracellular milieu;
iii) optionally, harvesting of the host cell(s);
iv) purification of the ApoE produced in step (ii);
v) optionally, lyophilization of the purified ApoE to obtain lyophilized ApoE;
vi) optionally, reconstitution of the lyophilized ApoE;
vii) optionally, separation of the ApoE according to their size;
viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL; most preferably to a concentration of about 500 µg/ml in TBS;
ix) inducing fibrillization of ApoE, optionally by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, EDTA, a buffer C, comprising NH₄HCO₃ (pH 7.8), and a buffer D, comprising NaCl, KCl, Na₂HPO₄, KH₂PO₄ (pH 7.4);
x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
xi) storage of the fibrillary ApoE at temperature in a range of about -20°C to about -80°C, preferably in a range of about -60°C to about -80°C, most preferably at about -80°C, until use.

7. The fibrillary ApoE for use according to any of the foregoing claims, wherein the method of treatment and/or prevention comprises administering the recombinant ApoE to a patient in an amount sufficient to induce an immune response; wherein, preferably, the immune response comprises the production of antibodies specifically binding to fibrillary ApoE, preferably fibrillary human ApoE4, by the patient.

8. An antigen-binding peptide specifically binding to fibrillary human ApoE, wherein the fibrillary human ApoE consists of an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 6, and SEQ ID No. 11, preferably SEQ ID No. 1.

9. The antigen-binding peptide according to claim 8, wherein the antigen-binding peptide is produced by a method comprising the steps of:
(a) generating fibrillary ApoE by a method comprising the steps:
i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, preferably SEQ ID No.1, SEQ ID No. 2, SEQ ID No. 3, SEQ ID No. 4, and SEQ ID No. 5;
ii) cultivation of the suitable host cell under conditions that favor the production of the ApoE for an appropriate time period, wherein, optionally, the ApoE is secreted into the extracellular milieu;
iii) optionally, harvesting of the host cell(s);
iv) purification of the ApoE produced in step (ii) and/or (iii);
v) optionally, lyophilization of the purified ApoE to obtain lyophilized ApoE;
vi) optionally, reconstitution of the lyophilized ApoE;
vii) optionally, separation of the ApoE according to their size;
viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL, most preferably to a concentration of about 500 µg/ml in TBS;
ix) inducing fibrillization of ApoE by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, and EDTA, a buffer C, comprising NH₄HCO₃ (pH 7.8), and a buffer D, comprising NaCl, KCl, Na₂HPO₄, KH₂PO₄ (pH 7.4);
x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
xi) storage of the fibrillary ApoE at temperature in a range of about -20°C to about -80°C, preferably in a range of about -60°C to about -80°C, most preferably at about -80°C, until use;
(b) administering the fibrillary ApoE, preferably ApoE in Buffer A, obtained in step (a) to a suitable animal host in an amount sufficient to induce an immune response; wherein, preferably, the immune response comprises the production of antibodies specifically binding to fibrillary ApoE, preferably fibrillary human ApoE, even more preferably to fibrillary human ApoE4;
wherein, the suitable animal host is selected from a group comprising rodents, mice, rats, rabbits, chickens, sheep, guinea pigs, goats, donkey, horses, and camelids; preferably selected from mice, rats, and rabbits; more preferably selected from mice and rats; even more preferably selected from wistar rats and CD1 mice;
wherein, preferably, the fibrillary ApoE is administered subcutaneously (s.c.) and/or intraperitoneally (i.p.);
wherein, optionally, the fibrillary ApoE is administered together with one or more further adjuvants, such as cytidine-phosphate-guanosine or incomplete Freud's adjuvant;
(c) keeping the animal host for a time period of about 5 weeks to about 20 weeks, preferably for about 8 weeks to about 17 weeks, more preferably for about 10 weeks to about 14 weeks, most preferably for about 12 weeks;
(d) optionally, during keeping in step (c), subsequent administering, preferably subcutaneously (s.c.) and/or intraperitoneally (i.p.) administering, of fibrillary ApoE about 1-7 days, preferably about 2-5 days, most preferably about 3 days before the end of keeping;
wherein, optionally, the fibrillary ApoE is administered together with one or more further adjuvants; wherein, preferably, the subsequent administering is without Freud's adjuvant;
(e) isolating immune cells from the spleen of immunized animals generated in steps (a) - (d) and fusion of said immune cells with an immortalized myeloma cell line, preferably myeloma cell line P3X63-Ag.653, to generate hybridoma cells;
(f) incubating generated hybridoma cells of step (e) for an appropriate time period followed by screening of the hybridoma cells to identify anti-fibrillary-ApoE antigen-binding peptide producing hybridoma cells;
(g) subcloning by limiting dilution of anti-fibrillary-ApoE antigen-binding peptide producing hybridoma cells to obtain stable monoclonal cell lines;
(h) cultivating the stable monoclonal cell lines obtained in step (g) under conditions suitable for the production of the anti-fibrillary-ApoE antigen-binding peptide;
(i) isolation, purification, and storage of the anti-fibrillary-ApoE antigen-binding peptide produced in step (h).

10. The antigen-binding peptide according to claim 8 or 9, wherein said antigen-binding peptide is a human antibody, a humanized antibody, or a chimeric-human antibody;
wherein, optionally, said antigen-binding peptide is an intact antibody, a substantially intact antibody, a Fab fragment, a F(ab')2 fragment, or a single chain Fv fragment.

11. A method for generating an antigen-binding peptide specifically binding to fibrillary ApoE, preferably fibrillary ApoE4, the method comprising the steps of:
(a) generating fibrillary ApoE by a method comprising the steps:
i) transfecting a suitable host cell for production of an ApoE with a nucleic acid encoding for an ApoE, preferably encoding for an ApoE comprising or consisting of an amino acid sequence which is at least 90% identical, more preferably at least 95% identical, even more preferably at least 98% identical, even more preferably at least 99% identical, optionally identical, to an amino acid sequence selected from a group comprising SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14, SEQ ID No. 15, preferably SEQ ID No.1, SEQ ID No. 2, SEQ ID No. 3, SEQ ID No. 4, and SEQ ID No. 5;
ii) cultivation of the suitable host cell under conditions that favor the production of the ApoE for an appropriate time period, wherein, optionally, the ApoE is secreted into the extracellular milieu;
iii) optionally, harvesting of the host cells;
iv) purification of the ApoE produced in step (ii) and/or (iii);
v) optionally, lyophilization of the purified ApoE to obtain lyophilized ApoE;
vi) optionally, reconstitution of the lyophilized ApoE;
vii) optionally, separation of the ApoE according to their size;
viii) adjusting the concentration of ApoE, preferably monomeric ApoE, of any of steps iii)-vii) to a concentration in the range of about 20 µg/mL to about 3000 µg/mL in TBS, preferably about 200 µg/mL to about 700 µg/mL, most preferably to a concentration of about 500 µg/ml in TBS;
ix) inducing fibrillization of ApoE by the addition of one or more further components; wherein, preferably, said further components are selected from a group comprising a buffer A, comprising Tris, NaCl, EDTA, KCl, Na₂HPO₄, and KH₂PO₄ (pH 7.4), a buffer B, comprising Tris, NaCl, EDTA, a buffer C, comprising NH₄HCO₃ (pH 7.8), and a buffer D, comprising NaCl, KCl, Na₂HPO₄, KH₂PO₄ (pH 7.4);
x) incubation at a temperature in a range of about 25°C to about 45°C, preferably in a range of about 30°C to about 40°C, more preferably in a range of about 35°C to about 40°C, most preferably at about 37°C, under constant agitation for a time period of about 1 week to about 5 weeks, preferably about 2 weeks to about 4 weeks, most preferably for about 3 weeks, thereby obtaining fibrillary ApoE;
xi) storage of the fibrillary ApoE at temperature in a range of about -20°C to about -80°C, preferably in a range of about -60°C to about -80°C, most preferably at about -80°C, until use;
(b) administering the fibrillary ApoE, preferably ApoE in Buffer A, obtained in step (a) to a suitable animal host in an amount sufficient to induce an immune response; wherein, preferably, the immune response comprises the production of antibodies specifically binding to fibrillary ApoE, preferably fibrillary human ApoE, even more preferably to fibrillary human ApoE4;
wherein, the suitable animal host is selected from a group comprising rodents, mice, rats, rabbits, chickens, sheep, guinea pigs, goats, donkey, horses, and camelids; preferably selected from mice, rats, and rabbits; more preferably selected from mice and rats; even more preferably selected from wistar rats and CD1 mice;
wherein, preferably, the fibrillary ApoE is administered subcutaneously (s.c.) and/or intraperitoneally (i.p.);
wherein, optionally, the fibrillary ApoE is administered together with one or more further adjuvants, such as cytidine-phosphate-guanosine and incomplete Freud's adjuvant;
(c) keeping the animal host for a time period of about 5 weeks to about 20 weeks, preferably for about 8 weeks to about 17 weeks, more preferably for about 10 weeks to about 14 weeks, most preferably for about 12 weeks;
(d) optionally, during keeping in step (c), subsequent administering, preferably subcutaneously (s.c.) and/or intraperitoneally (i.p.) administering, of fibrillary ApoE about 1-7 days, preferably about 2-5 days, most preferably about 3 days before the end of keeping;
wherein, optionally, the fibrillary ApoE is administered together with one or more further adjuvants; wherein, preferably, the subsequent administering is without Freud's adjuvant;
(e) isolating immune cells from the spleen of immunized animals generated in steps (a) - (d) and fusion of said immune cells with an immortalized myeloma cell line, preferably myeloma cell line P3X63-Ag.653, to generate hybridoma cells;
(f) incubating generated hybridoma cells of step (e) for an appropriate time period followed by screening of the hybridoma cells to identify anti-fibrillary-ApoE antigen-binding peptide producing hybridoma cells;
(g) subcloning by limiting dilution of anti-fibrillary-ApoE antigen-binding peptide producing hybridoma cells to obtain stable monoclonal cell lines;
(h) cultivating the stable monoclonal cell lines obtained in step (g) under conditions suitable for the production of the anti-fibrillary-ApoE antigen-binding peptide;
(i) isolation, purification, and storage of the anti-fibrillary-ApoE antigen-binding peptide produced in step (h).

12. An antigen-binding peptide generated by a method according to claim 11.

13. The antigen-binding peptide according to any of claims 8 to 10, or 12, for use in a method of treatment and/or prevention and/or diagnosis of a neurodegenerative disease in a patient in need thereof; wherein, preferably, the neurodegenerative disease is a neurodegenerative disease of the spectrum of central nervous system (CNS) diseases, more preferably, a neurodegenerative disease of the group of dementia conditions, even more preferably, a neurodegenerative diseases with an abnormal aggregation phenotype, even more preferably, an Alzheimer's disease such as early and late onset Alzheimer" s disease, Alzheimer" s disease including sporadic and/or familial cases, and Alzheimer's disease which occurs in conjunction with the presence of an ApoE4 allele;
wherein, optionally, the method of diagnosis of a neurodegenerative disease is a method of diagnosis of ApoE fibrillization in a biological sample derived from a patient; wherein, preferably, the biological sample is serum or cerebrospinal fluid; wherein, preferably, said method of diagnosis is performed during early stages of an Alzheimer's disease, such as preclinical Alzheimer's disease (biomarker positive), and the stage of mild cognitive impairment.

14. A composition, preferably a pharmaceutical composition, comprising a fibrillary ApoE for use as defined in any of claims 1 to 7, or an antigen-binding peptide according to any of claims 8 to 10, or 12.

15. The composition according to claim 14 for use in a method of treatment and/or prevention of a neurodegenerative disease in a patient in need thereof; wherein, preferably, the neurodegenerative disease is a neurodegenerative disease of the spectrum of central nervous system (CNS) diseases, more preferably, a neurodegenerative disease of the group of dementia conditions, even more preferably, a neurodegenerative diseases with an abnormal aggregation phenotype, even more preferably, an Alzheimer's disease such as early and late onset Alzheimer's disease, Alzheimer's disease including sporadic and/or familial cases, and Alzheimer" s disease which occurs in conjunction with the presence of an ApoE4 allele.

16. The fibrillary ApoE for use according to any of claims 1 to 7, or the antigen-binding peptide for use according to claim 13, or the composition for use according to claim 15, wherein the fibrillary ApoE, the antigen-binding peptide, or the composition is administered to a patient in need thereof;
wherein, preferably, the patient is a human or an animal, more preferably a human, even more preferably a human expressing ApoE4.
